# EUROPEAN PATENT SPECIFICATION

(11) **EP 1 448 760 B1**
(45) Date of publication and mention of the grant of the patent: **31.12.2008**
(21) Application number: 02792236.8
(22) Date of filing: 08.11.2002
(51) Int. Cl.: C12M 1/34, C07K 1/16, C07K 1/18, C07K 1/20, C07K 1/22, G01N 33/68

(54) **METHODS FOR MONITORING POLYPEPTIDE PRODUCTION AND PURIFICATION USING SURFACE ENHANCED LASER DESORPTION/IONIZATION MASS SPECTROMETRY**
VERFAHREN ZUR KONTROLLE VON POLYPEPTIDPRODUKTION UND -AUFREINIGUNG MIT OBERFLÄCHEN VERSTÄRKTER LASERDESORPTION/IONISATIONS MASSENSPEKTROMETRIE
PROCEDE DE SURVEILLANCE DE LA PRODUCTION ET DE LA PURIFICATION DE POLYPEPTIDES PAR SPECTROMETRIE DE MASSE A DESORPTION/IONISATION DE SURFACE PAR IMPACT LASER AMELIOREE

(30) Priority: 14.11.2001 US 335609 P; 16.04.2002 US 124626
(43) Date of publication of application: 25.08.2004
(73) Proprietor: Biorad Laboratories, Inc., Hercules, CA 94547 (US)
(72) Inventor: BOSCHETTI, Egisto, F-78290 Croissy sur Seine (FR); BRADBURY, Lisa, Newton Highlands, MA 02461 (US); DAVIES, Huw, Epsom Downs, Surrey KT1 74JP (GB); LOMAS, Lee, Foster City, CA 94404 (US); PHAM, Thang, T., Mountain View, CA 94040 (US); THULASIRAMAN, Vanitha, San Jose, CA 95148 (US); YIP, Tai-Tung, Cupertino, CA 95014 (US)
(74) Representative: Campbell, Patrick John Henry
(86) International application number: PCT/US2002/035971
(87) International publication number: WO 2003/042233

(56) References cited:
- US-A- 5 993 665
- US-B1- 6 377 721
- US-B1- 6 558 946
- AUSTEN B.M. ET AL: 'The use of Seldi ProteinChipTM Arrays to monitor production of Alzheimer's -amyloid in transfected cells' JOURNAL OF PEPTIDE SCIENCE vol. 6, no. 9, 14 September 2000, pages 459 - 469
- THULASIRAMAN VANITHA ET AL: 'Detection and identification of virulence factors in Yersinia pestis using SELDI ProteinChip(R) system' BIOTECHNIQUES vol. 30, February 2001, pages 428 - 432, XP008073159
- WINKLER MARTIN A. ET AL: 'Analysis of recombinant protein expression by MALDI-TOF mass spectrometry of bacterial colonies' BIOTECHNIQUES vol. 28, no. 5, May 2000, pages 890 - 895
- EASTERLING M.L.: 'Monitoring Protein Expression in Whole Bacterial Cells with MALDI Time-of-Flight Mass Spectrometry' ANAL. CHEM. vol. 70, 1998, pages 2704 - 2709
- JENSEN C. ET AL: 'Towards monitoring of protein purification by matrix-assisted laser desorption ionization mass spectrometry' INTERNATIONAL JOURNAL OF MASS SPECTROMETRY AND ION PROCESSES vol. 160, 1997, pages 339 - 356, XP004058841
- VILLANUEVA J. ET AL: 'Monitoring the expression and purification of recombinant proteins by MALDI-TOF mass spectrometry' ENZYME AND MICROBIAL TECHNOLOGY vol. 29, no. 1, 05 July 2001, pages 99 - 103

## Description

### BACKGROUND OF THE INVENTION

Bioprocessing technologies, particularly those involving mass cell culture techniques, are of increasing importance to the large-scale production of biological compounds, such as polypeptides with pharmaceutical, industrial, or agricultural applications. To illustrate, a DNA molecule encoding a target polypeptide (e.g., an antibiotic, a hormone, a cytokine, an enzyme, *etc*.) is typically cloned into an appropriate vector for expression in a suitable heterologous host cell, such as a mammalian, bacterial, or fungal cell. Transformed host cells are typically cultured in aqueous media that include nutrient sources of, *e*.*g*., carbon, hydrogen, phosphorus, potassium, nitrogen, or the like, electron acceptors (*e*.*g*., oxygen for aerobes, or nitrate or sulfate for anaerobes), and electron donors (*e*.*g*., carbohydrates, *etc*.) as appropriate to the specific host. Cells are generally cultured in bioreactors that are designed to support and maintain specific process conditions, including temperature, nutrient supply, and waste removal in, *e*.*g*., batch, fed batch, or continuous culture processes.

Active target polypeptides expressed in cell culture media are typically accompanied by numerous impurities, including degraded forms of the target in addition to non-target compounds that are separated to isolate the non-degraded target polypeptide in pure state. To this end, a number of separation and purification technologies are currently used. For example, if host cells secrete target molecules into the surrounding media, the cells are typically initially removed from the media using centrifugation or filtration, otherwise a cell disruption process is generally used to produce a cell lysate. Thereafter, targets are typically further purified using techniques, such as precipitation, chromatography, membrane separation, ultra-centrifugation, or the like. In spite of the separation performance of many of these methods, the final purified target biological formulation typically suffers from the presence of trace impurities, which are often the source of adverse effects, particularly when the formulation is used therapeutically. Examples of these impurities include small molecules, non-target proteins and peptides, nucleic acids, and endotoxins.

The nature and amount of impurities generally depend on the method selected to isolate the target biological. Moreover, starting biological materials are typically not consistent from one batch to another, and may contain impurities that differ according to a number of variables. For example, cell culture supernatants may contain proteins secreted as a reaction to specific or non-specific physiological stresses. The cellular metabolism may be slightly modified from one culture medium to another, which typically results in the presence of varied impurities in the different media despite the use of the same host strain in each batch.

Current techniques for the detection of trace impurities, such as electrophoresis, high performance liquid chromatography (HPLC), and certain immunoassays all suffer from significant limitations including, *inter alia*, poor selectivity, insufficient sensitivity, low throughput, unreliability, and/or high labor requirements. Accordingly, it is apparent that improved methods for the qualitative and/or quantitative detection of impurities and other components of, *e*.*g*., cell culture media or other complex mixtures are desirable. The present invention provides methods of monitoring the purity of a target protein at various stages of a purification process. The methods permit the protein of interest to be identified in a complex mixture and also to be distinguished from impurities with high sensitivity. These and other features of the invention will become apparent upon complete review of the following.

Methods for analysing target peptides in cell culture batches have been described in the art, for example in Austen et al (Journal of Peptide Science, 2000; vol.6(9), p459-469) and Thulasiraman et al (Biotechniques, 2001;vol.30, p428-432). Methods for monitoring protein purification using MALDI-MS techniques have also been described, for example in Jensen et al (International Journal of Mass Spectrometry and Ion Processes, vol.169, p339-356) and Villanueva et al (Enzyme and Microbial Technology, vol.29(1), p99-103).

### SUMMARY OF THE INVENTION

The present invention relates generally to the science and technologies of bioprocessing. In particular, the invention provides methods of monitoring the purification of target polypeptides from complex mixtures, such as cell culture media. The methods include generating surface enhanced laser desorption/ionization (SELDI) mass spectral profiles of biomolecular components in, *e*.*g*., a cell culture medium or a purified fraction of such a medium. The mass spectral profiles identify and/or quantify non-degraded target polypeptides and distinguish impurities with improved sensitivity relative to preexisting technologies. In addition, the methods described herein are optionally used to optimize large-scale purification processes. Surface enhanced laser desorption/ionization mass spectrometry is typically performed by exposing a sample to a substrate bound adsorbent to capture analyte molecules from the sample; and detecting the captured analyte molecules by laser desorption mass spectrometry. The substrate bound adsorbent can be a probe that is removably insertable into the mass spectrometer, which probe comprises a surface and the adsorbent attached to the surface. Unbound molecules can optionally be washed from the surface after exposure. Typically, desorption of biomolecular analytes is achieved with the assistance of energy absorbing molecules, referred to in some embodiments as "matrix."

The present invention relates to methods of monitoring purification of a target polypeptide from a mixture. The methods include (a) generating a first surface enhanced laser desorption/ionization mass spectral profile of biomolecular components in a mixture that includes the target polypeptide (*e*.*g*., a recombinant polypeptide, a naturally occurring polypeptide, or the like) and at least one contaminating biomolecule in which the first profile provides qualitative or quantitative detection of biomolecular components in the mixture. In certain embodiments, the mixture includes a cell culture medium from which cells have been removed, which cell culture medium comprises the target polypeptide. In other embodiments, the mixture includes a cell lysate. The detection generally includes determining the mass of the target polypeptide and the at least one contaminating biomolecule. The methods also include (b) subjecting the target polypeptide to a purification process by removing at least a portion of at least one contaminating biomolecule from the mixture to provide a purer mixture that includes the target polypeptide, and (c) generating a second surface enhanced laser desorption/ionization mass spectral profile of biomolecular components in the purer mixture. In addition, the methods include (d) comparing the first and second profiles to determine a qualitative or quantitative difference between biomolecular components in the mixture and the purer mixture, thereby monitoring the purification of the target polypeptide. The methods optionally further include identifying one or more contaminating biomolecules in the mixture or the purer mixture.

The present invention provides methods of purifying a target polypeptide. The methods include (a) identifying purification conditions by: (i) contacting a mixture comprising the target polypeptide with a plurality of substrate-bound adsorbents (*e*.*g*., chromatographic adsorbents, biospecific adsorbents, or the like), (ii) washing each of the adsorbents with a different eluant to allow selective binding of polypeptides in the mixture to the adsorbents, (iii) generating a surface enhanced laser desorption/ionization mass spectral profile of biomolecular components in the mixture in which a surface enhanced laser desorption/ionization mass spectral profile provides qualitative or quantitative detection of biomolecular components in the mixture, and (iv) identifying (1) a wash condition under which the target polypeptide is adsorbed to the adsorbent and contaminating polypeptides are eluted from the adsorbent and (2) a wash condition under which the target polypeptide is eluted from the adsorbent. The substrate-bound adsorbents are generally in the form of a probe. Further, the wash conditions typically include different parameters selected from one or more of, *e*.*g*., a salt concentration, a detergent concentration, a pH, a buffering capacity, an ionic strength, a water structure characteristic, a detergent type, a hydrophobicity, a dielectric constant, a concentration of at least one solvent (*e*.*g*., an organic solvent, *etc*.), a concentration of at least one solute, or the like. The methods also include (b) contacting a batch (*e*.*g*., at least about 1000 liters, *etc*.) of the mixture with a chromatographic medium that includes the adsorbent and washing the chromatographic medium with an identified wash condition under which the target polypeptide is adsorbed to the adsorbent and contaminating polypeptides are eluted from the adsorbent. The methods further include (c) washing the chromatographic medium with an identified wash condition under which the target polypeptide is eluted from the adsorbent, and (d) collecting the eluted target polypeptide.

### BRIEF DESCRIPTION OF THE DRAWINGS

Figure 1 is a flow chart that schematically shows steps involved in an embodiment of a method of monitoring purification of a target polypeptide from a mixture.

Figure 4 is a flow chart that schematically shows steps involved in an embodiment of a method of purifying a target polypeptide.

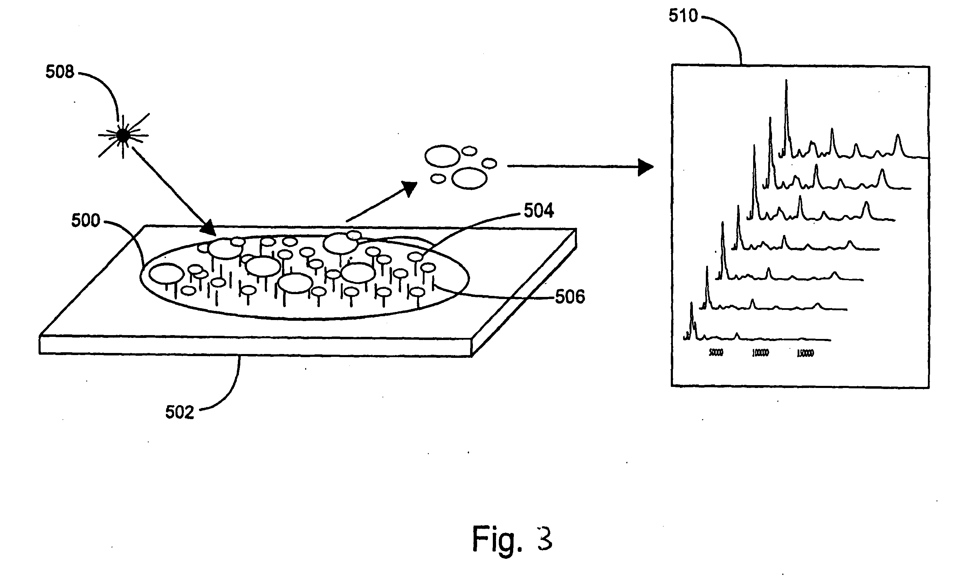

Figure 3 schematically depicts a surface enhanced laser desorption/ionization assay of a cell culture medium sample to detect a presence of a target polypeptide.

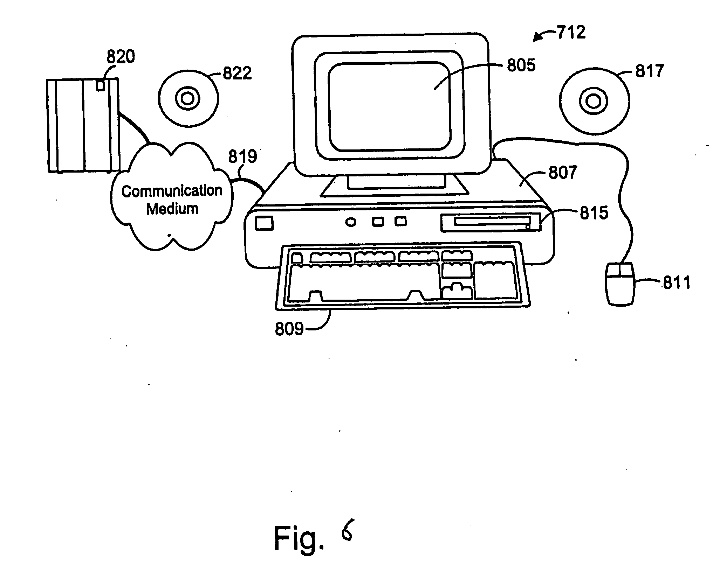

Figure 6 schematically depicts a surface enhanced laser desorption/ionization assay for monitoring the purification of a target polypeptide from a mixture.

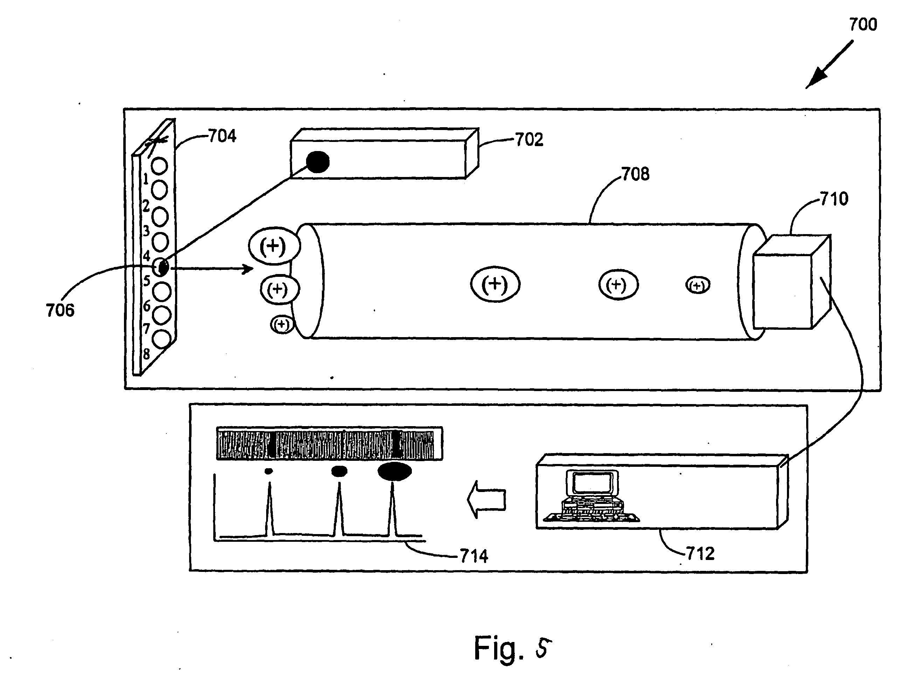

Figure 5 schematically illustrates a surface enhanced laser desorption/ionization time-of-flight mass spectrometry system.

Figure 6 is schematically illustrates a representative example information appliance or digital device in which various aspects of the present invention may be embodied.

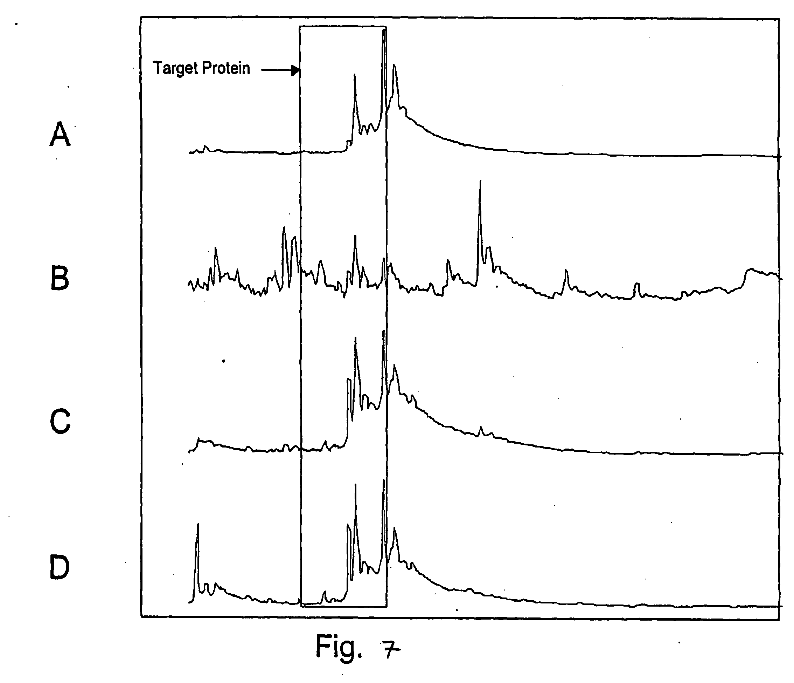

Figure 7A-D are mass spectral traces showing detected components from cell culture media using H4 and IMAC3 ProteinChip® arrays.

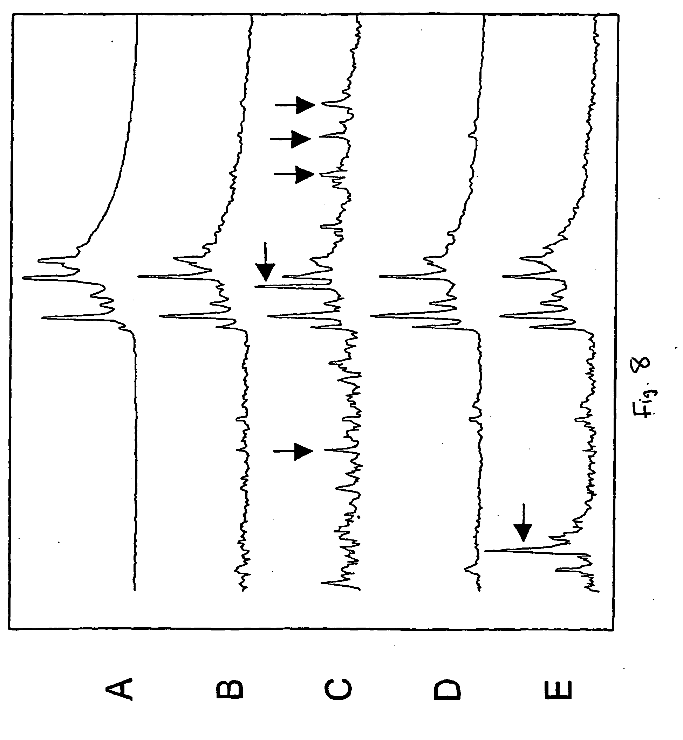

Figures 8A-E are mass spectral traces showing detected components from different lots of culture media using H4 ProteinChip® arrays.

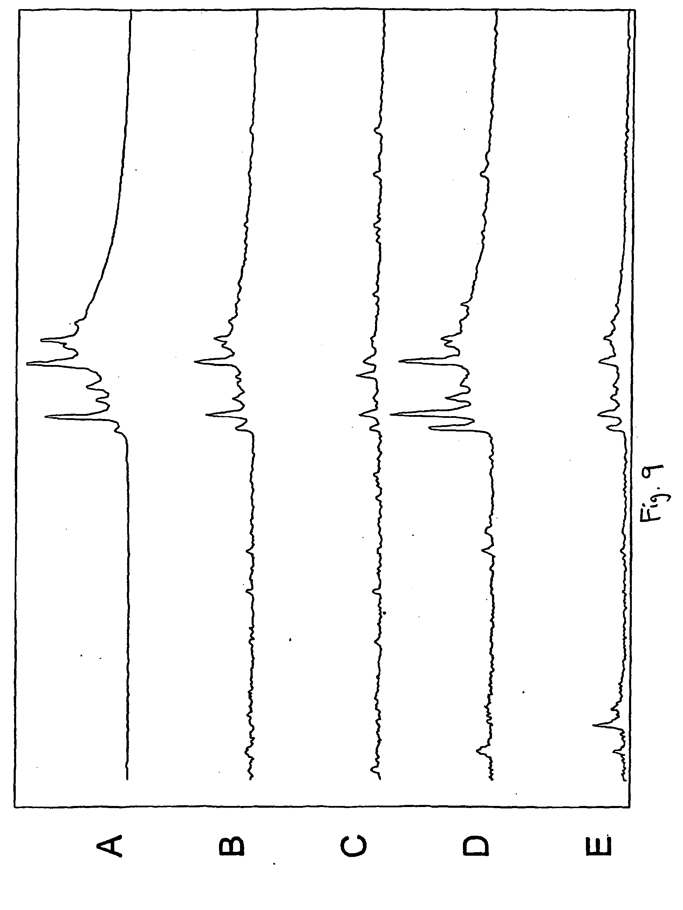

Figures 9A-E are mass spectral traces showing detected components from a quantitative analysis of different lots of culture media using H4 ProteinChip® arrays.

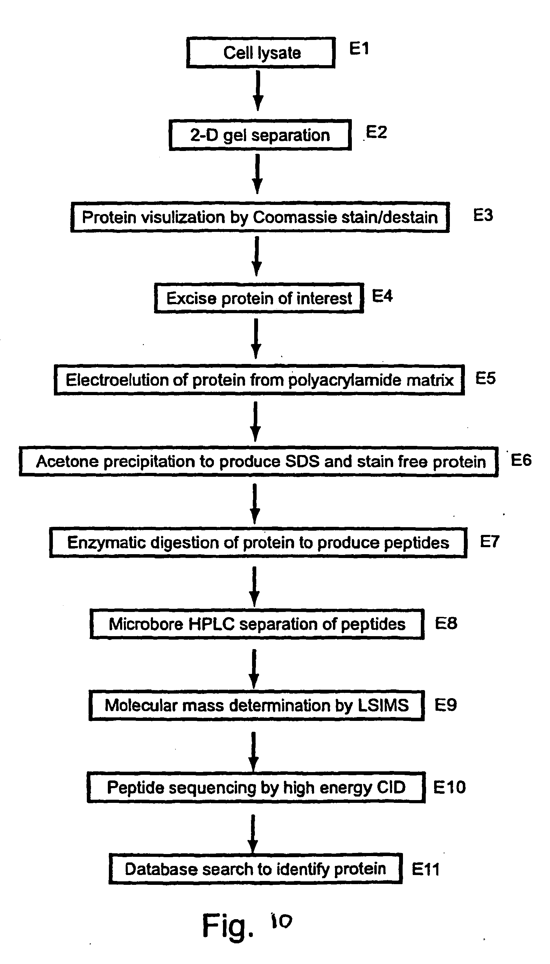

Figure 10 is a flow chart that schematically shows steps involved in a procedure for identifying proteins from 2-D gels.

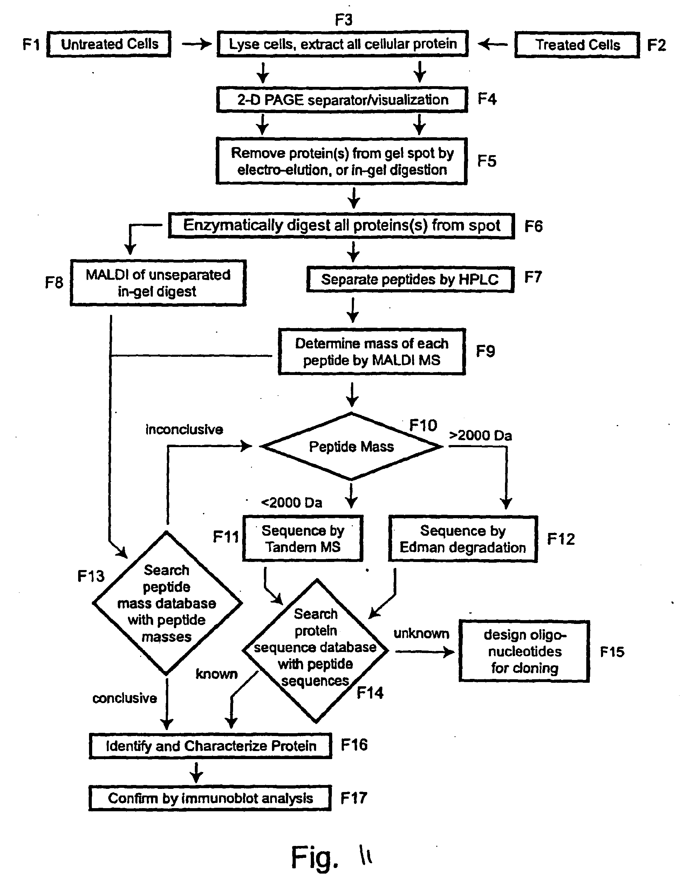

Figure 11 is a flow diagram that schematically shows various steps involved in a procedure for identifying proteins from 2-D gels.

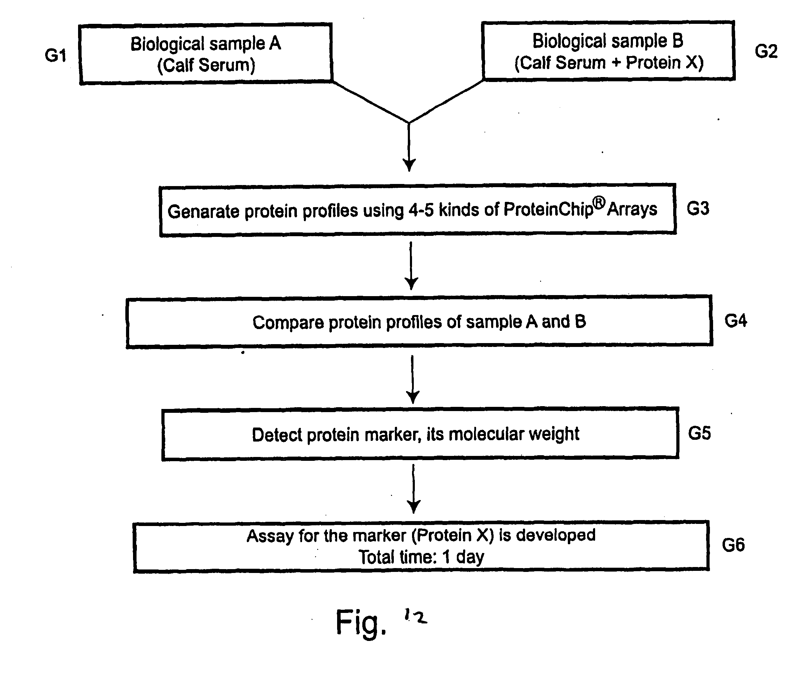

Figure 12 is a flow chart that schematically shows steps involved in a protein profiling procedure.

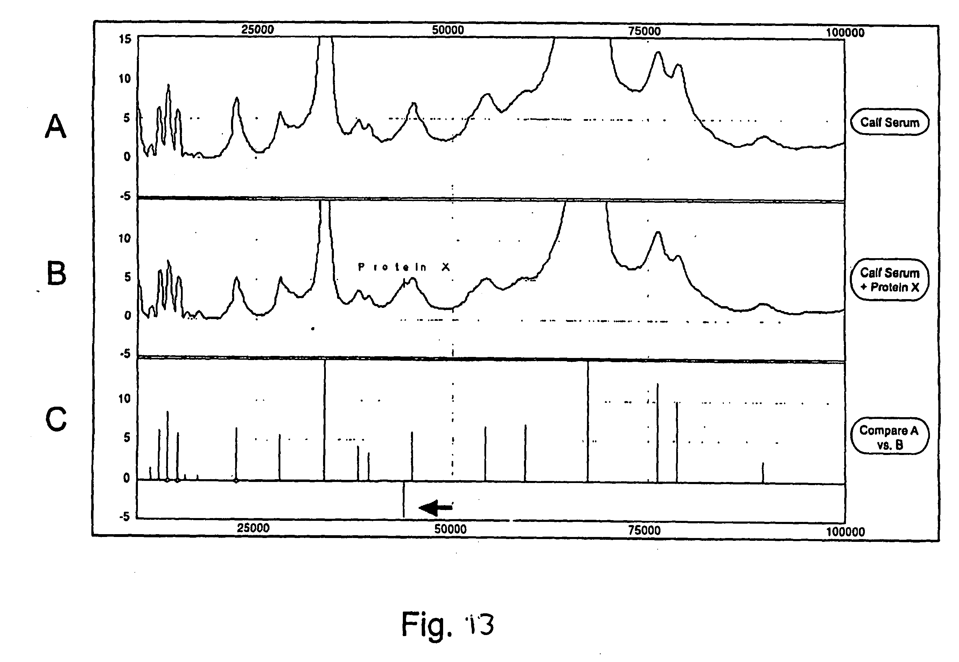

Figures 13A-C are mass spectral traces showing detected protein profiles on NP ProteinChip® arrays after being washed and a difference map.

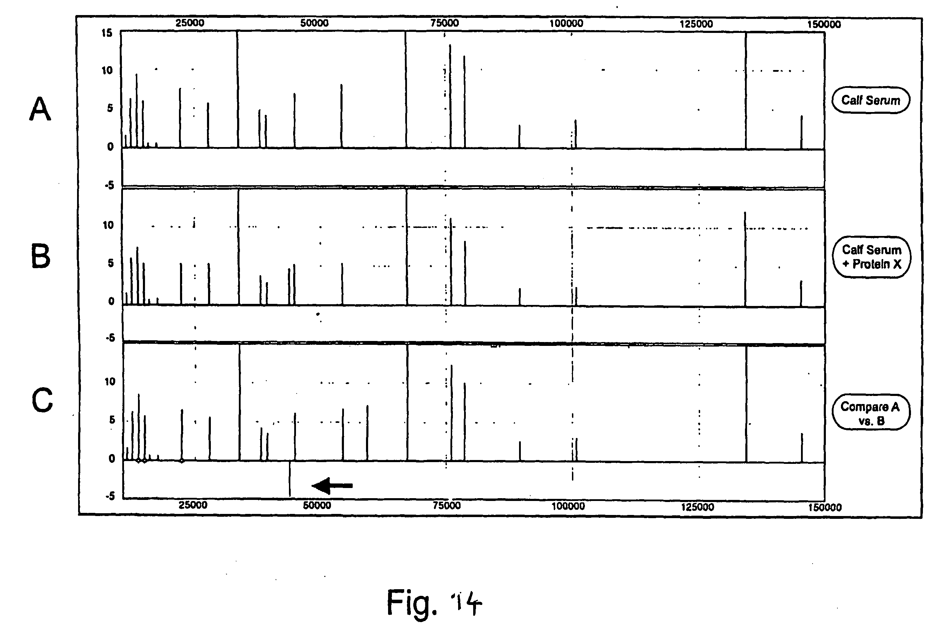

Figures 14A-C are mass spectral traces showing detected protein profiles on NP ProteinChip® arrays after being washed and a difference map.

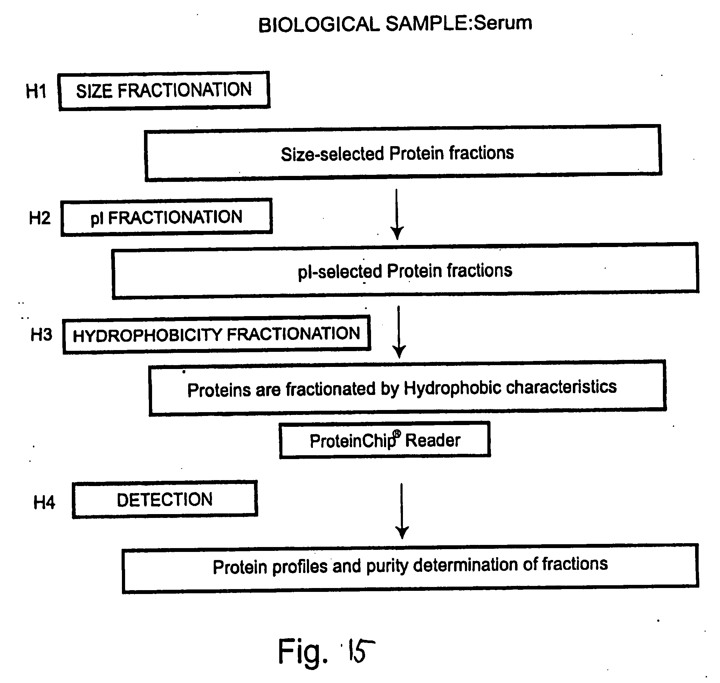

Figure 15 is a flow diagram that schematically shows a general protein fractionation scheme for biological samples.

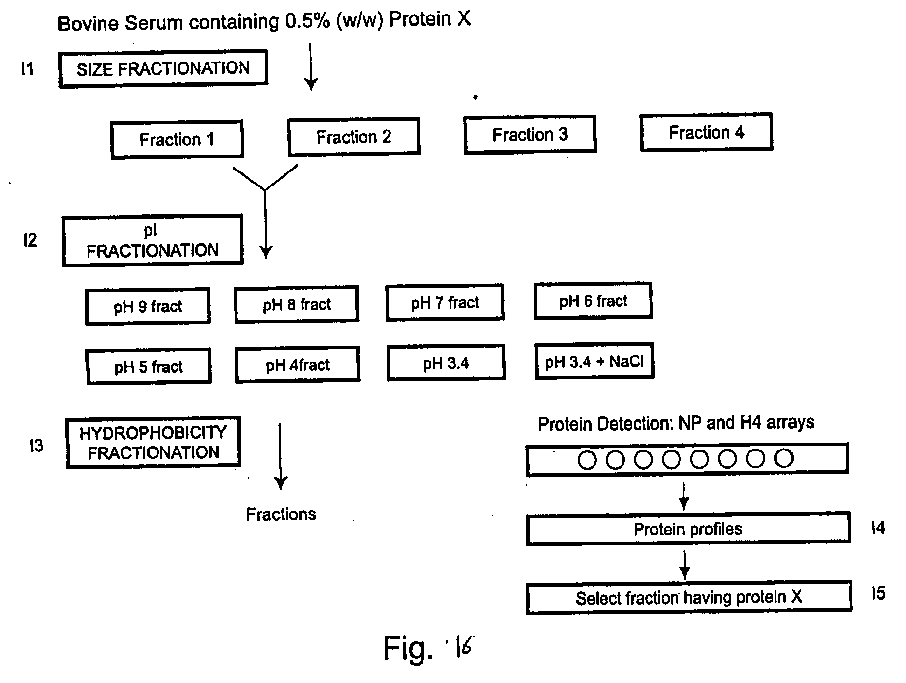

Figure 16 is a flow diagram that schematically shows various steps in a micropurification of a target protein from bovine serum.

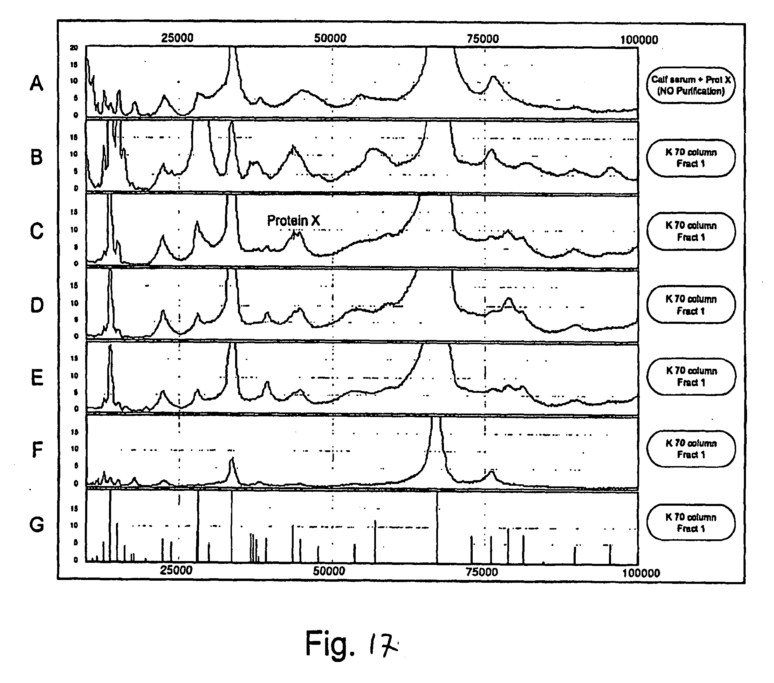

Figures 17A-G are mass spectral traces showing detected protein profiles from a fractionation assay of bovine serum on size-selection spin columns.

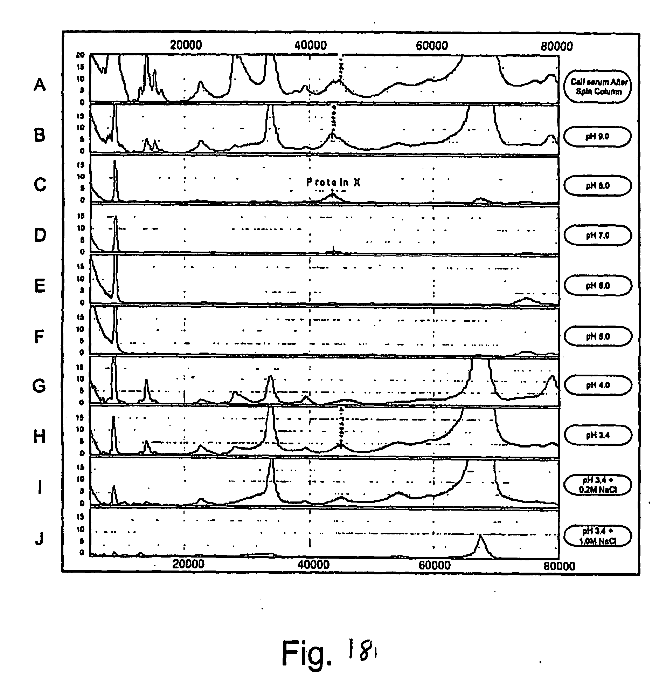

Figures 18A-J are mass spectral traces showing detected protein profiles from a fractionation assay of bovine serum on anion exchange spin columns.

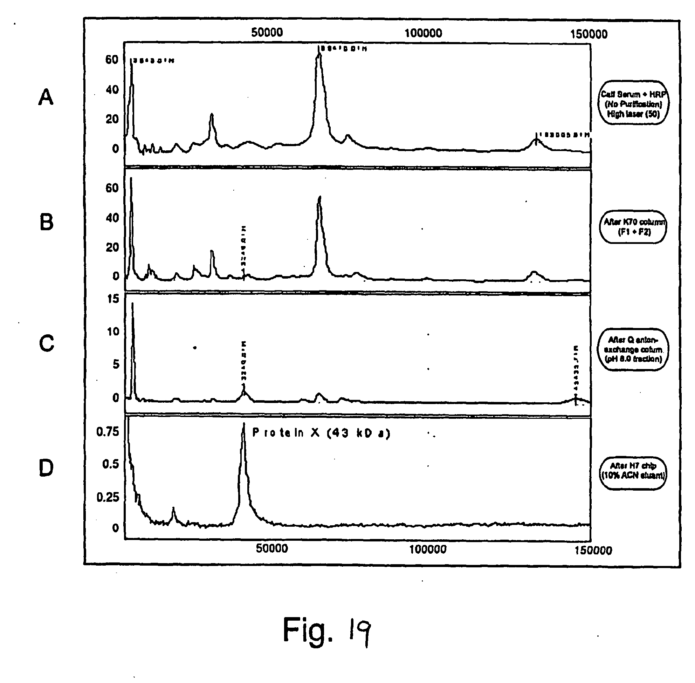

Figures 19A-D are mass spectral traces showing a progression of purification of a target protein from bovine serum.

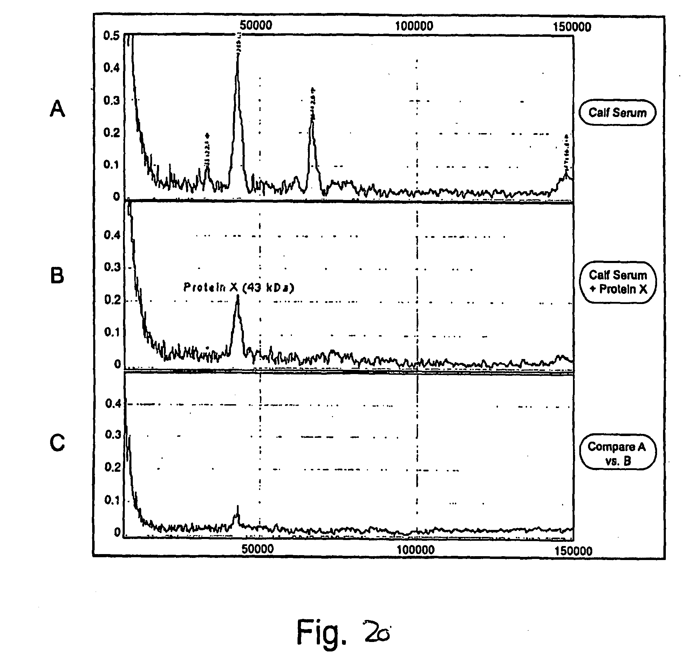

Figures 20A-C are mass spectral traces showing purification of a target protein on C8 agarose spin columns.

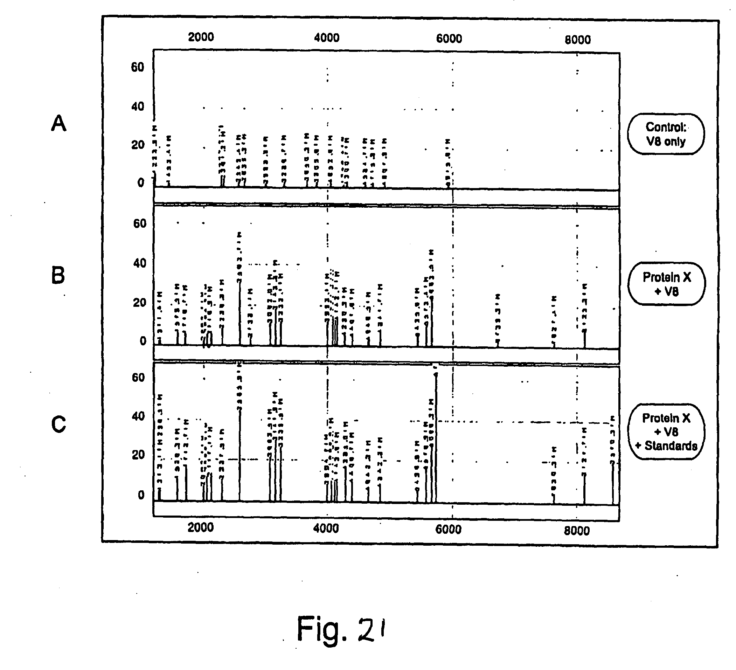

Figures 21A-C are mass spectral traces of a purified target protein digested with V8 (Glu C) endopeptidase which fingerprint the target protein.

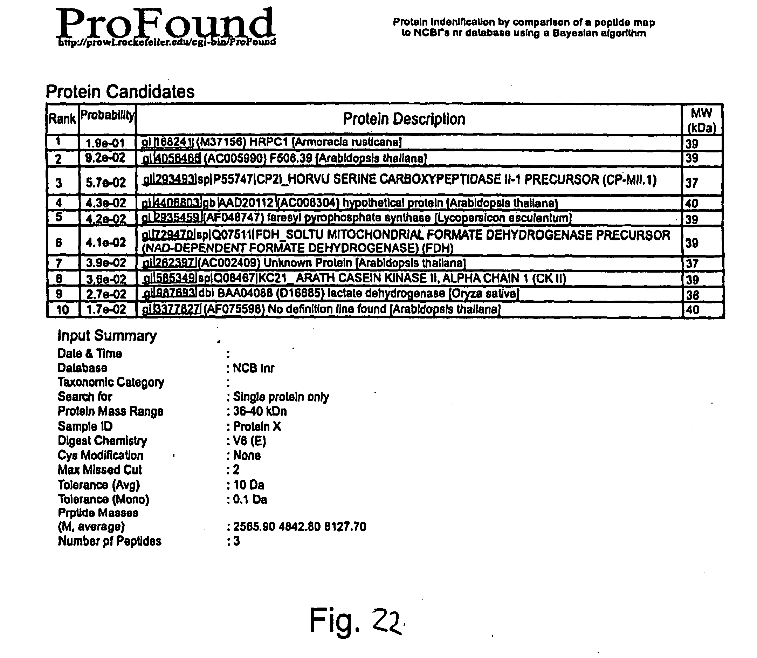

Figure 22 shows a display screen for a ProFound database search using a purified target protein digested with V8 (Glu C) endopeptidase.

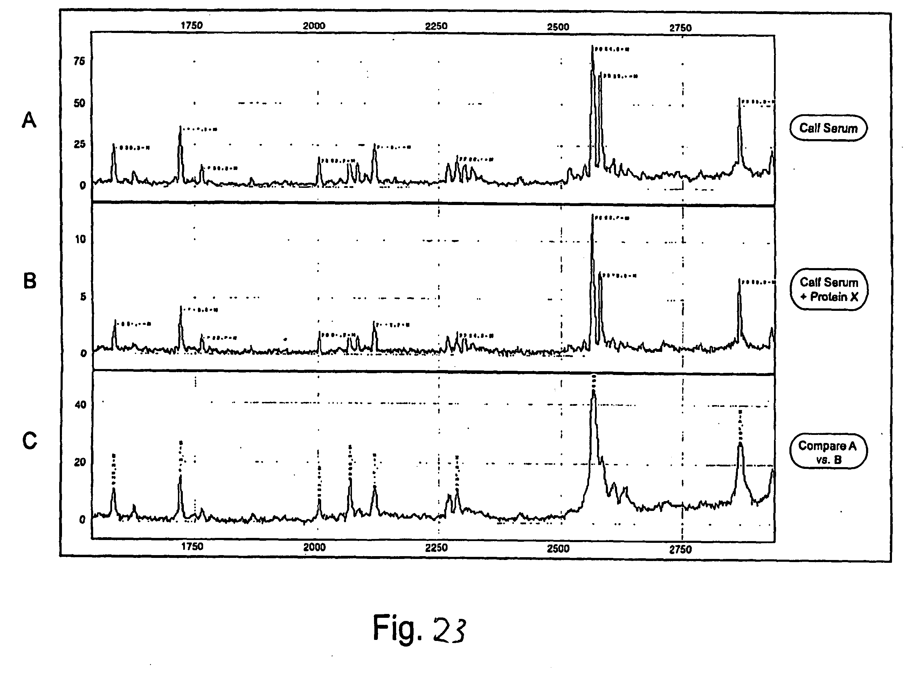

Figures 23A-C are mass spectral traces showing detected peptide fragments produced by V8 endopeptidase digestion of an HRP sample.

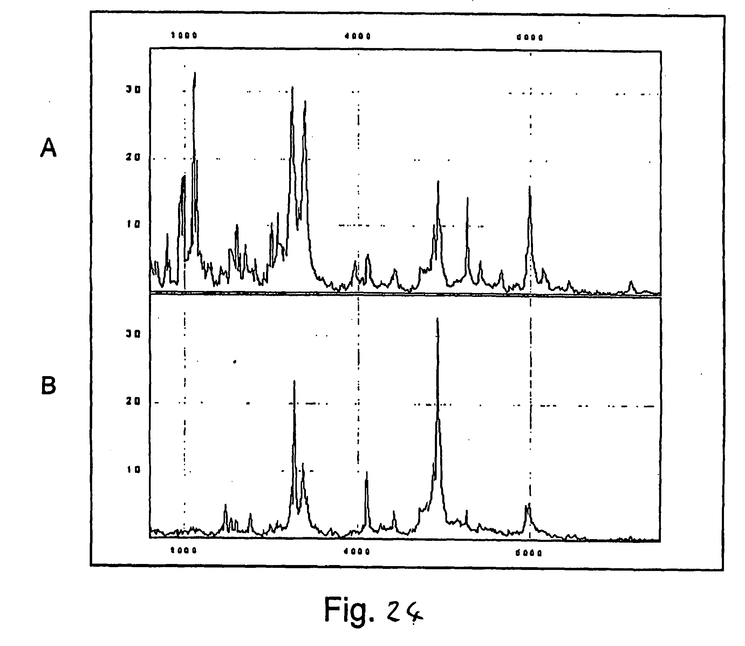

Figures 24 A and B are mass spectral traces showing detected peptide fragments from a tryptic digest of a target protein under different conditions.

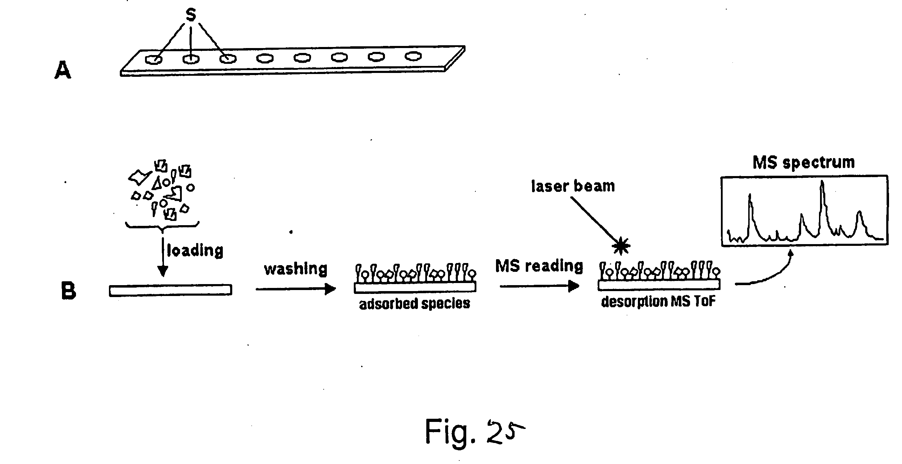

Figures 25 A and B schematically further depict a method of using ProteinChip® array surfaces for selective adsorption of proteins and SELDI-TOF MS analysis of captured protein species. More specifically, Figure 25A schematically shows an array with 8 spots (S) for sample loading. Figure 25B schematically illustrates a process of loading, washing, and desorbing captured protein species prior to SELDI-TOF MS analysis.

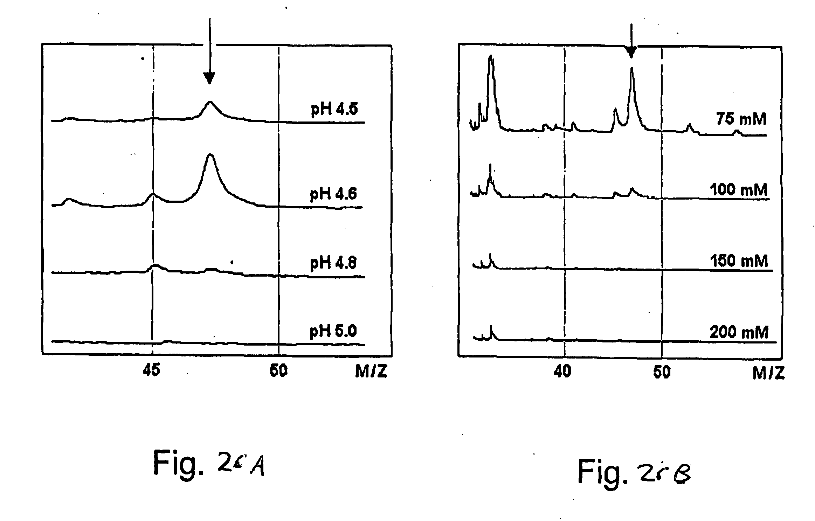

Figures 26 A and B are mass spectral traces (abscissa - molecular weight (Daltons); ordinate - relative intensity) showing detected protein profiles from a WCX 2 ProteinChip® array surface loaded with a crude, clarified extract of *E. coli* containing an Fab antibody fragment. In particular, Figure 26A provides mass spectral traces of proteins retained at different pH's with the Fab fragment represented by the arrow. Figure 26B provides mass spectral traces of proteins retained at different sodium chloride concentrations in a 50 mM acetate, 5 mM citrate buffer at pH 4.6 with the Fab fragment represented by the arrow.

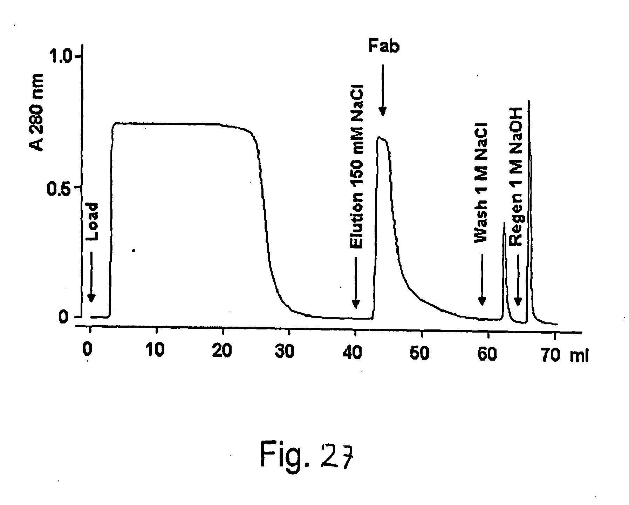

Figure 27 is a chromatogram obtained from the separation of crude *E. coli* extract on CM Zirconia sorbent. Twenty-three ml of crude material was loaded onto the column (0.3 cm ID x 10 cm) previously equilibrated with a 50 mM acetate, 5 mM citrate buffer at pH 4.6. The flow rate through the column was 300 cm/hour. Elution of the Fab fragment was obtained by increasing sodium chloride concentration up to 150 mM in the equilibration buffer. The column was finally washed with a 1 M sodium chloride solution prior to regeneration with 1 M sodium hydroxide.

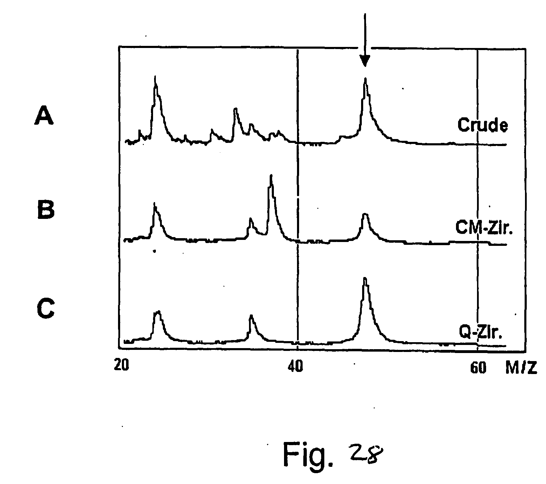

Figures 28A-C are mass spectral traces (abscissa - molecular weight (Daltons); ordinate - relative intensity) showing detected protein profiles obtained using an NP2 (normal phase) ProteinChip® array and SELDI-TOF MS. Figure 28A is a mass spectral trace showing a detected protein profile from a crude extract of *E. coli* containing an Fab antibody fragment. Figure 28B is a mass spectral trace showing a detected protein profile from eluate obtained from a CM Zirconia chromatography column through which crude extract of *E. coli* containing an Fab antibody fragment was run. Figure 28C is a mass spectral trace showing a detected protein profile from flow-through obtained from a Q Ceramic HyperD® F chromatography column through which crude extract of *E. coli* containing an Fab antibody fragment was run. In each of these figures, the detected Fab fragment is indicated by the arrow.

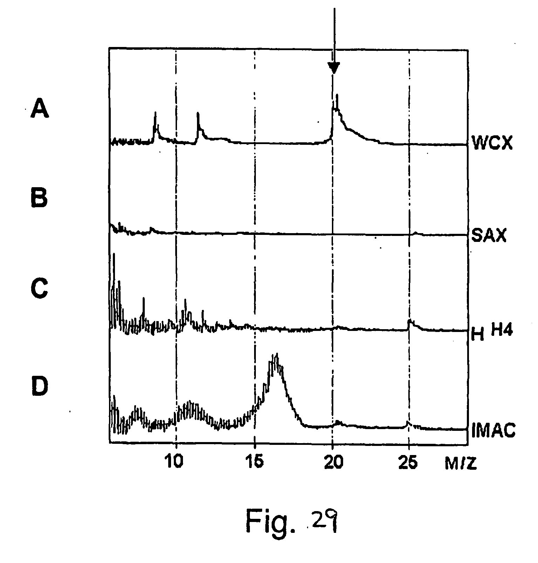

Figures 29A-D are mass spectral traces (abscissa - molecular weight (Daltons); ordinate - relative intensity) showing detected protein profiles obtained using SELDI-TOF MS and different ProteinChip® Array surfaces loaded with a *Pichia pastoris* cell culture supernatant containing endostatin. More specifically, Figure 29A is a mass spectral trace obtained using weak cationic exchange (WCX 2 ProteinChip® array) in 50 mM acetate/5 mM citrate buffer at pH 4.5. Figure 29B is a mass spectral trace obtained using strong anionic exchange (SAX 2 ProteinChip® array) in 50 mM Tris-HCl buffer at pH 8. Figure 29C is a mass spectral trace obtained using a hydrophobic interaction surface (H4 ProteinChip® array) in 50 mM Tris-HCl buffer, 1000 mM sodium chloride at pH 7.5. Figure 29D is a mass spectral trace obtained using a chelated surface with Cu⁺² (IMAC 3 ProteinChip® array) in 20 mM phosphate buffer, 500 mM sodium chloride at pH 7.0. Endostatin is indicated by the arrow in each of these figures.

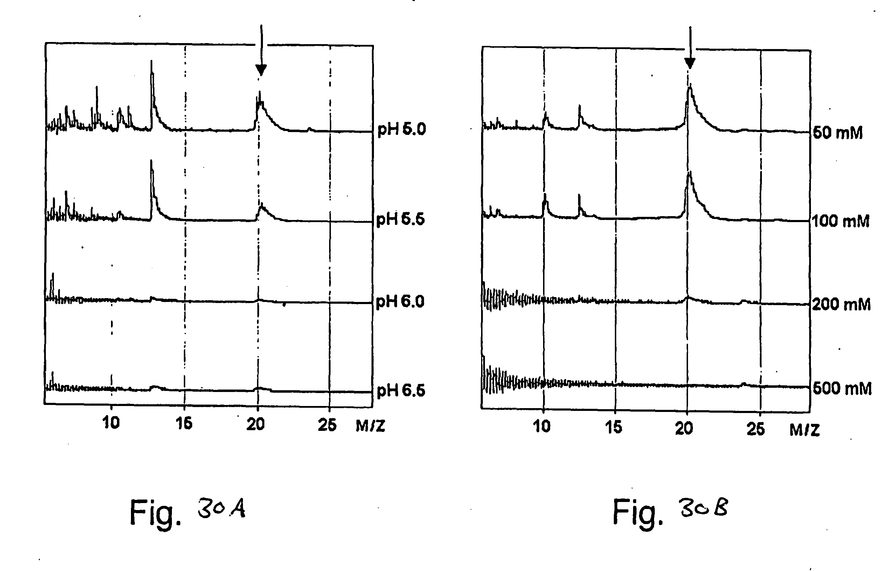

Figures 30 A and B are mass spectral traces (abscissa - molecular weight (Daltons); ordinate - relative intensity) showing detected protein profiles obtained using SELDI-TOF MS with a WCX 2 ProteinChip® array loaded with a *Pichia pastoris* cell culture supernatant containing endostatin. The retained proteins shown in the four traces of Figure 30A were obtained from retentate chromatography analyses in 50 mM acetate, 5mM citrate buffer, each at the different pH levels indicated. In contrast, Figure 30B shows mass spectral traces of proteins retained at different sodium chloride concentrations in a 50 mM acetate/5 mM citrate buffer, pH 5. Endostatin is indicated by the arrow in each of these figures.

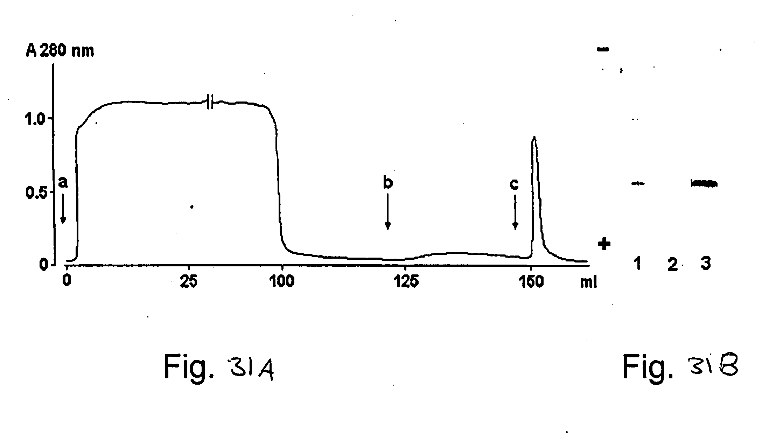

Figure 31 A is a chromatogram obtained from the separation of crude *Pichia pastoris* culture supernatant on CM Zirconia sorbent. The separation involved loading 80 ml of crude material onto a column (0.3 cm ID x 10 cm) previously equilibrated with a 50 mM acetate, 5 mM citrate, 50 mM sodium chloride buffer at pH 5. The flow rate through the column was 300 cm/hour. Elution of endostatin was obtained by increasing the sodium chloride concentration in two steps. The first step was done using 200 mM sodium chloride in the equilibration buffer (a). The second step was performed with 800 mM sodium chloride in the same buffer (b).

Figure 31 B is an electropherogram obtained from electrophoretic separations of different fractions involved in the chromatographic analysis described with respect to Figure 31 A. As shown, crude supernatant was separated in lane 1, non-adsorbed fraction (flow-through) was separated in lane 2, and an elution fraction at 800 mM sodium chloride was separated in lane 3.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd Ed. 1994), The Cambridge Dictionary of Science and Technology (Walker ed., 1988), The Glossary of Genetics, 5th Ed., R. Rieger et al. (eds.), Springer Verlag (1991), and Hale & Marham, The Harper Collins Dictionary of Biology (1991). As used herein, the following terms have the meanings ascribed to them unless specified otherwise.

"Substrate" or "probe substrate" refers to a solid phase onto which an adsorbent can be provided (*e*.*g*., by attachment, deposition, or the like). "Surface feature" refers to a particular portion, section, or area of a substrate or probe substrate onto which adsorbent can be provided.

"Surface" refers to the exterior or upper boundary of a body or a substrate.

"Adsorbent" refers to any material capable of adsorbing an analyte (e.g., a target polypeptide). The term "adsorbent" is used herein to refer both to a single material ("monoplex adsorbent") (*e*.*g*., a compound or functional group) to which the analyte is exposed, and to a plurality of different materials ("multiplex adsorbent") to which the analyte is exposed. The adsorbent materials in a multiplex adsorbent are referred to as "adsorbent species." For example, a surface feature on a probe substrate can comprise a multiplex adsorbent characterized by many different adsorbent species (*e*.*g*., ion exchange materials, metal chelators, antibodies, or the like), having different binding characteristics. Substrate material itself can also contribute to adsorbing an analyte and may be considered part of an "adsorbent." A "biomolecular interaction adsorbent" or "biospecific adsorbent," such as an affinity adsorbent, a polypeptide, an enzyme, a receptor, an antibody (*e*.*g*., a monoclonal antibody, *etc*.), or the like, typically has higher specificity for a target analyte than a "chromatographic adsorbent," which includes, *e*.*g*., an anionic adsorbent, a cationic adsorbent, a hydrophobic interaction adsorbent, a hydrophilic interaction adsorbent, a metal-chelating adsorbent, or the like.

"Adsorption," "capture," or "retention" refers to the detectable binding between an adsorbent and an analyte (*e*.*g*., a target polypeptide) either before or after washing with an eluant (selectivity threshold modifier) or a washing solution.

"Eluant," "wash," or "washing solution" refers to an agent that can be used to mediate adsorption of an analyte to an adsorbent. Eluants and washing solutions also are referred to as "selectivity threshold modifiers." Eluants and washing solutions can be used to wash and remove unbound or non-captured materials from the probe substrate surface.

"Specific binding" refers to binding that is mediated primarily by the basis of attraction of an adsorbent for a designated analyte (*e*.*g*., a target polypeptide). For example, the basis of attraction of an anionic exchange adsorbent for an analyte is the electrostatic attraction between positive and negative charges. Therefore, anionic exchange adsorbents engage in specific binding with negatively charged species. The basis for attraction of a hydrophilic adsorbent for an analyte is hydrogen bonding. Therefore, hydrophilic adsorbents engage in specific binding with electrically polar species or the like.

"Resolve," "resolution," or "resolution of analyte" refers to the detection of at least one analyte in a sample. Resolution includes the detection and differentiation of a plurality of analytes in a sample by separation and subsequent differential detection. Resolution does not require the complete separation of an analyte from all other analytes in a mixture. Rather, any separation that allows the distinction between at least two analytes suffices.

"Probe" refers to a device that, when positionally engaged in an interrogatable relationship to an ionization source, *e*.*g*., a laser desorption/ionization source, and in concurrent communication at atmospheric or subatmospheric pressure with a detector of a gas phase ion spectrometer, can be used to introduce ions derived from an analyte into the spectrometer. As used herein, the "probe" is typically reversibly engageable (*e*.*g*., removably insertable) with a probe interface that positions the probe in an interrogatable relationship with the ionization source and in communication with the detector. A probe will generally comprise a substrate comprising a sample presenting surface on which an analyte is presented to the ionization source. "Ionization source" refers to a device that directs ionizing energy to a sample presenting surface of a probe to desorb and ionize analytes from the probe surface into the gas phase. The preferred ionization source is a laser (used in laser desorption/ionization), in particular, nitrogen lasers, Nd-Yag lasers and other pulsed laser sources. Other ionization sources include fast atoms (used in fast atom bombardment), plasma energy (used in plasma desorption) and primary ions generating secondary ions (used in secondary ion mass spectrometry).

"Gas phase ion spectrometer" refers to an apparatus that detects gas phase ions. In the context of this invention, gas phase ion spectrometers include an ionization source used to generate the gas phase ions. Gas phase ion spectrometers include, for example, mass spectrometers, ion mobility spectrometers, and total ion current measuring devices.

"Gas phase ion spectrometry" refers to a method that includes employing an ionization source to generate gas phase ions from an analyte presented on a sample presenting surface of a probe and detecting the gas phase ions with a gas phase ion spectrometer.

"Mass spectrometer" refers to a gas phase ion spectrometer that measures a parameter which can be translated into mass-to-charge ratios of gas phase ions. Mass spectrometers generally include an inlet system, an ionization source, an ion optic assembly, a mass analyzer, and a detector. Examples of mass spectrometers are time-of-flight, magnetic sector, quadrapole filter, ion trap, ion cyclotron resonance, electrostatic sector analyzer and hybrids of these.

"Mass spectrometry" refers to a method that includes employing an ionization source to generate gas phase ions from an analyte presented on a sample presenting surface of a probe and detecting the gas phase ions with a mass spectrometer.

"Laser desorption mass spectrometer" refers to a mass spectrometer which uses laser as a means to desorb, volatilize, and ionize an analyte.

"Desorption ionization" refers to generating ions by desorbing them from a solid or liquid sample with a high-energy particle beam (*e*.*g*., a laser). Desorption ionization encompasses various techniques including, *e*.*g*., surface enhanced laser desorption, matrix-assisted laser desorption, fast atom bombardment, plasma desorption, or the like.

"Surface-enhanced laser desorption/ionization" or "SELDI" is a method of gas phase ion spectrometry in which the surface of substrate which presents the analyte to the energy source plays an active role in the desorption and ionization process. The SELDI technology is described in, *e*.*g*., U.S. patent 5,719,060 (Hutchens and Yip).

"Surface-enhanced laser desorption/ionization mass spectral profile" refers to a profile indicating signal as a function of time-of-flight or a derivative of time-of-flight, such as mass, acquired through the performance of surface enhanced laser desorption/ionization mass spectrometry.

"Retentate chromatography" is a process for fractionating biomolecules on a solid phase adsorbent and analyzing the fractionated molecules by SELDI. Retentate chromatography is described in, *e*.*g*., International Publication WO 98/59360 (Hutchens and Yip).

"Matrix-assisted laser desorption/ionization" or "MALDI" refers to an ionization source that generates ions by desorbing them from a solid matrix material with a pulsed laser beam.

"Detect" refers to identifying the presence, absence or amount of the object to be detected.

"Biomolecule," "biomolecular component," or "bioorganic molecule" refers to an organic molecule typically made by living organisms. This includes, for example, molecules that includes nucleotides, amino acids, sugars, fatty acids, steroids, nucleic acids, polypeptides, peptides, peptide fragments, carbohydrates, lipids, and combinations of these (*e*.*g*., glycoproteins, ribonucleoproteins, lipoproteins, or the like).

"Biological material" refers to any material derived from an organism, organ, tissue, cell or virus. This includes biological fluids such as saliva, blood, urine, lymphatic fluid, prostatic or seminal fluid, milk, *etc.,* as well as extracts of any of these, e.g., cell extracts, cell culture media, fractionated samples, or the like.

"Energy absorbing molecule" or "EAM" refers to a molecule that absorbs energy from an ionization source in a mass spectrometer thereby enabling desorption of analyte, such as a target polypeptide, from a probe surface. Depending on the size and nature of the analyte, the energy absorbing molecule can optionally be used. Cinnamic acid derivatives, sinapinic acid ("SPA"), cyano hydroxy cinnamic acid ("CHCA"), and dihydroxybenzoic acid are frequently used as energy absorbing molecules in laser desorption of bioorganic molecules. *See,* U.S. Patent No. 5,719,060 to Hutchens and Yip for additional description of energy absorbing molecules.

The terms "polypeptide," "peptide," and "protein" are used interchangeably herein to refer to a polymer of amino acid residues. The terms apply to amino acid polymers in which one or more amino acid residues are analogs, derivatives or mimetics of corresponding naturally occurring amino acids, as well as to naturally occurring amino acid polymers. For example, polypeptides can be modified or derivatized, *e*.*g*., by the addition of carbohydrate residues to form glycoproteins. The terms "polypeptide," "peptide," and "protein" include glycoproteins, as well as non-glycoproteins.

A "target polypeptide" or "target biological" refers to a protein to be identified.

"Polypeptide production" refers to the in vivo or in vitro expression of a polynucleotide that encodes a target polypeptide, or to the synthesis of a target polypeptide, *e*.*g*., using a solid-phase synthesis technique or another approach known in the art.

### DETAILED DISCUSSION OF THE INVENTION

### I. INTRODUCTION

The present invention relates to methods of monitoring, optimizing, and scaling-up various stages of assorted bioprocesses. In particular, the invention provides methods to simultaneously detect multiple biomolecular components, including protein impurities separated from target polypeptides expressed by recombinant living systems. The protein impurities typically include essentially any host cell protein, proteins from cell culture media, released proteinaceous affinity ligands, or the like. The methods include analyzing target polypeptides and/or impurities by mass spectrometry using interactive surfaces (ProteinChip® Arrays) with bound adsorbents of the biomolecular components.

More specifically, the methods utilize surface enhanced laser desorption ionization (SELDI) associated with a mass spectrometrical analysis to resolve mixtures of proteins or other biomolecules for detection and identification with significantly improved sensitivity relative to many preexisting technologies. For example, this analytical tool is used according to the methods described herein to detect impurities, such as host cell proteins from purified biologicals expressed during cell culture processes. The selectivity of the methods depends, at least in part, on the nature of the chemical derivatization of the probe surface to which the sample is applied, which is generally similar to adsorbents used to perform, *e*.*g*., "normal phase" or "reverse phase" HPLC. In certain embodiments, samples are pre-fractionated, *e*.*g*., using various fractionation techniques (*e*.*g*., chromatography, *etc*.) prior to being applied to a probe surface, *e*.*g*., to simplify the interpretation of results when the number of impurities is expected to be high. After a sample is deposited on a probe surface, selected biomolecules are adsorbed to the surface in a chosen buffer as a result of interactions with the adsorbent. Following an optional wash to eliminate all non-desired components, such as salts, energy absorbing molecules are applied to the probe surface. Upon desorption and ionization from the probe by an incident laser beam, biomolecular components are analyzed by mass spectrometry. Components of the mixture are evidenced by differences in detected molecular weights.

Omitted.

The invention provides methods of monitoring target protein purification. In particular, differences in impurity profiles (*e*.*g*., residual host cell proteins, degraded target polypeptides, *etc*.) can result from an under-optimized purification process. However, when a purification process is well controlled, these differences may relate to the underlying cell culture medium. For example, the nature of cell culture media may also include foreign proteins or other biomolecules that are equally to be removed from the biological of interest and therefore their absence from the final purified product is to be checked. In contrast, when analyzing protein-free cell culture media, any detected non-target proteins are ostensibly generated by the host cells in the culture. Furthermore, the number of protein contaminants, as well as their amounts, are both important parameters for assessing the level of purity of a target biological, being indicative of the efficacy and robustness of the separation process.

This invention also provides methods of monitoring contaminants coming from chromatographic sorbent degradation. This relates to the release of ligands and their hydrolysis products. A typical example is Protein A used as ligand for resins capable to separate antibodies. Protein A can be released during antibody fractionation as whole and as fragments as a result of breakdown by the presence in the feed stock of traces of proteases. This invention can be used to monitor Protein A contamination of the immunoglobulin.

In particular, the methods of the present invention provide numerous advantages over many other analytical techniques, such as electrophoresis, HPLC, 2-dimensional electrophoresis, capillary electrophoresis, and immunoassays for the analysis of impurities in separated biological samples from expression systems. These advantages are briefly described below.

### Compared to One-Dimensional Electrophoresis:

Unlike the methods of the present invention, even the best electrophoretic systems are limited by poor sensitivity. For example, to detect proteins in an electrophoregram, protein bands are typically labeled with specific dyes, which do not have the same affinity for all proteins, such that the color intensity of the band may not be directly proportional to the amount of protein in the band. In many cases, small proteins cannot be detected in electrophoretic systems. In contrast, the methods of the present invention detect proteins independent of abilities to interact with other chemical compounds. Instead, detection is effected directly such that all proteins are evidenced in a similar manner. In addition, electrophoresis is a laborious and time consuming technique, whereas the methods of the invention provide results within a short period of time to provide an essentially on-line detection tool.

### Compared to Two-Dimensional Electrophoresis:

This analytical method typically includes separating proteins in a first dimension according to the isoelectric points of individual proteins. Proteins are also separated in a second dimension in the presence of SDS according to molecular mass differences. As with one-dimensional electrophoresis, separated proteins are typically indirectly detected by being stained with selected dyes (*e*.*g*., Coomassie blue) to produce a map of spots. Unlike the present invention, this process is time-consuming (*e*.*g*., typically taking over a day to produce a map) and the interpretation of the resulting data is difficult. Also, the loading capacity of this technique is low, which acts to further limit sensitivity. In particular, this technique does not detect low abundance proteins, or small proteins and peptides (*e*.*g*., below 10-20 kDa). The sensitivity of this technique is additionally limited, because proteins with very low and very high isoelectric points are generally not separated in the first dimension.

### Compared to HPLC:

A large variety of separation properties optionally form the basis of this method. Typically, reverse phase HPLC is used for analytical purposes. However, other separation principles, such as ion exchange, molecular sieving, normal phase, and affinity are also optionally used. The efficiency of separation is generally related to the size of particles in the solid-phase within the column. To increase the separation efficiency very small particles are typically used with an attendant increase in pressure inside the column. Expensive instruments are designed to make HPLC separations under high pressure. In addition to the expense, separation times are also relatively long. Further, separation conditions are typically designed on a case-by-case basis, which adds to the time and complexity of a given separation. Moreover, complex mixtures are generally only poorly separated by HPLC, and the technique is limited to relatively small proteins. HPLC suffers also from some low level of sensitivity depending on the type of molecules to detect.

### Compared to Capillary Electrophoresis:

In certain circumstances capillary electrophoresis provides better separation efficiency than HPLC, but is capable of handling only very low loads, which limits the detectability of low abundance proteins. This is a significant limitation especially when the detection of trace amounts of impurities is the objective, such as in target protein preparations intended for therapeutic use.

### Compared to Enzyme-Linked Immunosorbent Assays (ELISA):

Unlike the methods described herein, when immunoassays, such as ELISA are used to detect trace impurities, the target impurities must typically be known in advance, because specific antibodies against each single target impurity are typically used. ELISA does not detect impurities for which antibodies are not present. Accordingly, this method is generally not readily adapted to detect a large diversity of protein impurities, especially when impurities vary from one sample to another. Also, the production of specific antibodies is time consuming and laborious. In addition, antibodies are generally not exactly the same from one batch to another, which gives rise to different levels of sensitivity in the final assay. Further, immunoassays typically do not detect all protein fragments generated by, e.g., proteolysis, because the fragments may not include the particular amino-acid sequence recognized by the antibodies to the protein. In contrast, the present invention detects biomolecular components based on differences in molecular masses.

### II. TARGET POLYPEPTIDE SOURCES

For purposes of clarity of illustration, the methods of the present invention are described primarily in the context of processes that involve target polypeptides. However, it will be appreciated by those of skill in the art to which this invention pertains that the methods of the invention may be applied or adapted to essentially any production and/or purification process in which target chemical species are to be resolved from, e.g., complex mixtures of non-target or otherwise contaminating compounds.

Essentially any polypeptide can be qualitatively and/or quantitatively detected using the methods described herein. Common repositories for known protein sequences or nucleic acids that encode such proteins include, *e*.*g*., GenBank®, EMBL, DDBJ, NCBI, or the like. Target sequences of interest typically include, *e*.*g*., various pharmaceutically, agriculturally, industrially, or otherwise commercially relevant proteins. Certain exemplary target polypeptides of interest optionally include, *e*.*g*., hormones, cytokines, immunoglobulins, enzymes, receptors, antigens, regulation factors, protein carriers, or the like. Additional sequences corresponding to these and to other potential targets are known in the art and are readily obtainable by known methods.

In particular, the target polypeptides are optionally naturally occurring or the ultimate products of forced molecular diversification processes, such as nucleic acid recombination, mutagenesis, or other techniques known in the art. For example, additional details regarding recombination protocols, such as sexual and assembly PCR, which are optionally used to generate nucleic acids that encode the target polypeptides monitored according to the methods of the invention, are provided in, *e*.*g*., Crameri et al. (1996) "Improved green fluorescent protein by molecular evolution using DNA shuffling" Nature Biotechnology 14:315-319 and Stemmer (1994) "Rapid evolution of a protein in vitro by DNA shuffling" Nature 370:389-391. Additional details relating to nucleic acid mutagenesis techniques, such as site-directed mutagenesis, cassette mutagenesis, error-prone PCR, or the like are described in, *e*.*g*., Dale et al. (1996) "Oligonucleotide-directed random mutagenesis using the phosphorothioate method" Methods Mol. Biol. 57:369-374, Caldwell et al. (1992) "Randomization of genes by PCR mutagenesis" PCR Methods Applic. 2:28-33, Smith (1985) "In vitro mutagenesis" Ann. Rev. Genet. 19:423-462, Kunkel et al. (1987) "Rapid and efficient site-specific mutagenesis without phenotypic selection" Methods in Enzymol. 154, 367-382, and Wells et al. (1985) "Cassette mutagenesis: an efficient method for generation of multiple mutations at defined sites" Gene 34:315-323. Forced molecular diversification processes are also generally described in, *e*.*g*., Smith and Jones (Eds.), DNA Recombination and Repair, Oxford University Press, Inc. (2000), McPherson (Ed.), Directed Mutagenesis: A Practical Approach, Oxford University Press, Inc. (1991), Trower (Ed.), In Vitro Mutagenesis Protocols, Vol. 57, Humana Press (1996), and Sankaranarayanan, Protocols in Mutagenesis, Vol. 1, Elsevier Science (2001). Kits for recombination, mutagenesis, and other aspects of library construction are also commercially available. For example, kits are available from, *e*.*g*., Clonetech Laboratories, Stratagene, Epicentre Technologies, New England Biolabs, Pharmacia Biotech, and Promega Corp.

General texts which describe additional molecular biological techniques useful herein, including library construction, transformation, host organism selection (*e*.*g*., eukaryotic or prokaryotic cells, transgenic plants or animals, *etc*.), cell culture, and other aspects of molecular biology include Berger and Kimmel, Guide to Molecular Cloning Techniques, Methods in Enzymology, Vol. 152, Academic Press, Inc. (Berger), Sambrook et al., Molecular Cloning - A Laboratory Manual (2nd Ed.), Vol. 1-3, Cold Spring Harbor Laboratory (1989) (Sambrook), and Current Protocols in Molecular Biology, F.M. Ausubel et al. (Eds.), Current Protocols, a joint venture between Greene Publishing Associates, Inc. and John Wiley & Sons, Inc. (supplemented through 2000) (Ausubel). Methods of transducing cells, including plant and animal cells, with nucleic acids are generally available, as are methods of expressing proteins encoded by such nucleic acids. In addition to Berger, Ausubel and Sambrook, useful general references for culturing animal cells include Freshney, Culture of Animal Cells, a Manual of Basic Technique, 3rd Ed., Wiley-Liss (1994)(Freshney), Humason, Animal Tissue Techniques, 4th Ed., W.H. Freeman and Company (1979), and Ricciardelli et al., In Vitro Cell Dev. Biol. 25:1016-1024 (1989). References for plant cell cloning, culture and regeneration include Payne et al., Plant Cell and Tissue Culture in Liquid Systems, John Wiley & Sons, Inc. (1992)(Payne) and Gamborg and Phillips (Eds.), Plant Cell, Tissue and Organ Culture; Fundamental Methods Springer Lab Manual, Springer-Verlag (1995)(Gamborg).

Mass cell culture techniques are widely known in the science of bioprocessing. In particular, additional details relating to cell culture, culture media, and culture equipment are provided in, *e*.*g*., Fiechter (Ed.), Advances in Biochemical Engineering-Biotechnology: Bioprocess Design and Control, Springer-Verlag, Inc. (1993), Kargi, Bioprocess Engineering, 2nd, Prentice Hall (2001), Buckland (Ed.), Cell Culture Engineering, Kluwer Academic Publishers (1995), Doran , Bioprocess Engineering Principles, Academic Press, Inc. (1995), Vieth , Bioprocess Engineering: Kinetics, Mass Transport, Reactors, and Gene Expression, John Wiley & Sons, Inc. (1994), Butler, Animal Cell Culture and Technology: The Basics, Oxford University Press, Inc. (1998), and Davis (Ed.), Basic Cell Culture: A Practical Approach, 2nd, Oxford University Press, Inc. (2001).

The samples used in the methods of this invention are also optionally derived from other biological material sources. This includes body fluids such as blood, serum, saliva, urine, prostatic fluid, seminal fluid, seminal plasma, lymph, lung/bronchial washes, mucus, feces, nipple secretions, sputum, tears, or the like. It also includes extracts from biological samples, such as cell lysates, *etc*. For example, cell lysate samples are optionally derived from, *e*.*g*., primary tissue or cells, cultured tissue or cells, or the like. Biological samples are optionally collected according to any known technique, such as venipuncture, biopsy, or the like. Many references are available for the culture and production of cells, including cells of bacterial, plant, animal (especially mammalian) and archebacterial origin. *See e*.*g*., Ausubel, Berger, Freshney, Doyle, Payne, and Gamborg, *supra*. The specific exemplary target protein sources listed herein are offered to illustrate but not to limit the present invention. Additional sources of protein samples are known in the art and are readily obtainable.

### III. METHODS FOR MONITORING PURIFICATION OF A TARGET POLYPEPTIDE AND FOR PROCESS SCALE-UP

The present invention provides methods of monitoring the purification of a target polypeptide and for scaling-up purification processes. For example, the methods are optionally used to make a proper follow-up for the removal of impurities all along the purification process (*e*.*g*., cell host proteins, ligands released from affinity columns, *etc*.). In addition to SELDI-TOF, the use of a classical MALDI-TOF is also a optionally used where no selective adsorption of proteins on a probe surface occurs. SELDI and MALDI analyses are described in greater detail below. Further, the described methods are optionally used to track possible released materials from purification processes, *e*.*g*., protein ligands used in chromatography, such as protein A (purification of immunoglobulins G) and immunosorbents involving antibodies as ligands (separation of antigens).

Polypeptides are optionally recovered and purified from cell cultures by any of a number of methods well known in the art, including electrophoresis, chromatography, precipitation, dialysis, filtration, and/or centrifugation. More specifically, purification techniques, such as ultra-centrifugation, ammonium sulfate or ethanol precipitation, acid extraction, ion exchange chromatography, high performance liquid chromatography, size exclusion chromatography, phosphocellulose chromatography, hydrophobic interaction chromatography, affinity chromatography (*e*.*g*., using any of the tagging systems), hydroxylapatite chromatography, and/or lectin chromatography are optionally used. Preferably, the sample is in a liquid form from which solid materials (*e*.*g*., cellular debris, *etc*.) have been removed. In addition to the references noted herein, a variety of purification methods are well known in the art, including, *e*.*g*., those set forth in Sandana, Bioseparation of Proteins, Academic Press, Inc. (1997), Bollag et al., Protein Methods, 2nd Ed., Wiley-Liss (1996), Walker, The Protein Protocols Handbook, Humana Press (1996), Harris and Angal, Protein Purification Applications: A Practical Approach, IRL Press (1990), Harris and Angal (Eds.), Protein Purification Methods: A Practical Approach, IRL Press (1989), Scopes, Protein Purification: Principles and Practice, 3rd Ed., Springer Verlag (1993), Janson and Ryden, Protein Purification: Principles, High Resolution Methods and Applications, 2nd Ed., Wiley-VCH (1998), Walker, Protein Protocols on CD-ROM, Humana Press (1998), Satinder Ahuja ed., Handbook of Bioseparations, Academic Press (2000), and the references cited therein. Sample fractionation techniques, which optionally utilize certain of these purification techniques, *e*.*g*., to prepare samples for SELDI mass spectral profiling are described further below.

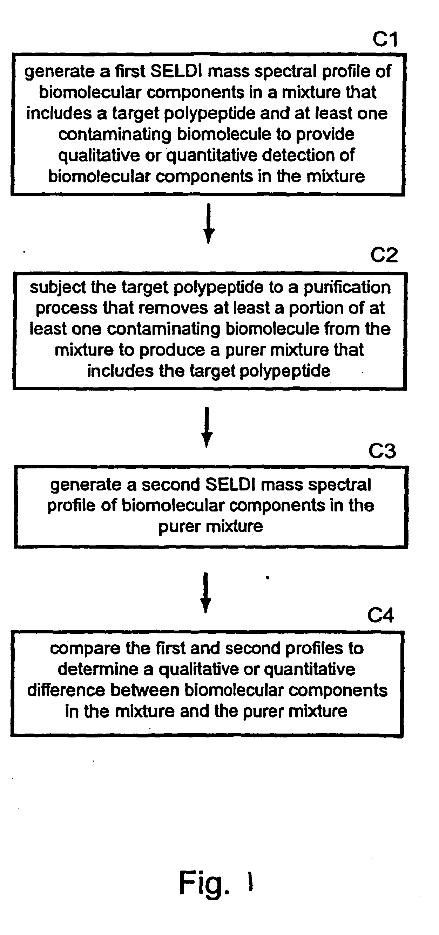

Figure 1 is a flow chart that schematically shows steps involved in an embodiment of a method of monitoring purification of a target polypeptide from a mixture. As shown, **C1** includes generating a first SELDI mass spectral profile of biomolecular components in a mixture that includes a target polypeptide and at least one contaminating biomolecule to provide qualitative or quantitative detection of biomolecular components in the mixture. In **C2,** the method includes subjecting the target polypeptide to a purification process, such as those referred to herein or otherwise known in the art, that removes at least a portion of at least one contaminating biomolecule from the mixture to produce a purer mixture that includes the target polypeptide. In addition, **C3** includes generating a second SELDI mass spectral profile of biomolecular components in the purer mixture. Finally, **C4** includes comparing the first and second profiles to determine a qualitative or quantitative difference between biomolecular components in the mixture and the purer mixture. As described herein, the methods typically include culturing cells under conditions to produce the target polypeptide and collecting the mixture from the cultured cells. In certain embodiments, the cultured cells secrete the target polypeptide into a cell culture medium during target polypeptide production and, *e*.*g*., the mixture includes the cell culture supernatant, which has been separated from the host cell population. If the target polypeptide is not secreted into the surrounding medium, the cultured cells are typically lysed prior collecting the mixture, *e*.*g*., using essentially any cell harvesting process known in the art.

In certain embodiments, the methods include determining a quantitative difference in purity of the target polypeptide in the mixture and the purer mixture in which purity is a measure of relative amounts of the target polypeptide and the at least one contaminating biomolecule. In some embodiments, the methods include determining a qualitative difference between the target polypeptide in the mixture and in the purer mixture in which the qualitative difference is a measure of the target polypeptide and degraded forms of the target polypeptide. In still other embodiments, the methods include determining a qualitative difference in the target polypeptide between the mixtures in which the qualitative difference includes a difference in the target polypeptide and a modified form of the target polypeptide in which the modification (*e*.*g*., modified in vivo or in vitro) is selected from, *e*.*g*., glycosylation, phosphorylation, lipidation, enzymatic breakdown, labeling, polypeptide aggregation, or the like. As an additional option, the methods include determining a quantitative or qualitative difference between contaminating biomolecular components in the first and second profiles.

In some embodiments, the methods further include subjecting the target polypeptide to at least one additional purification process to provide at least one subsequent mixture, and generating a subsequent SELDI mass spectral profile of biomolecular components in the at least one subsequent mixture. These embodiments also typically include comparing the subsequent SELDI mass spectral profile to another SELDI mass spectral profile (*e*.*g*., the first and/or second mass spectral profiles, *etc*.) to determine a qualitative or quantitative difference between biomolecular components in the subsequent mixture and another mixture.

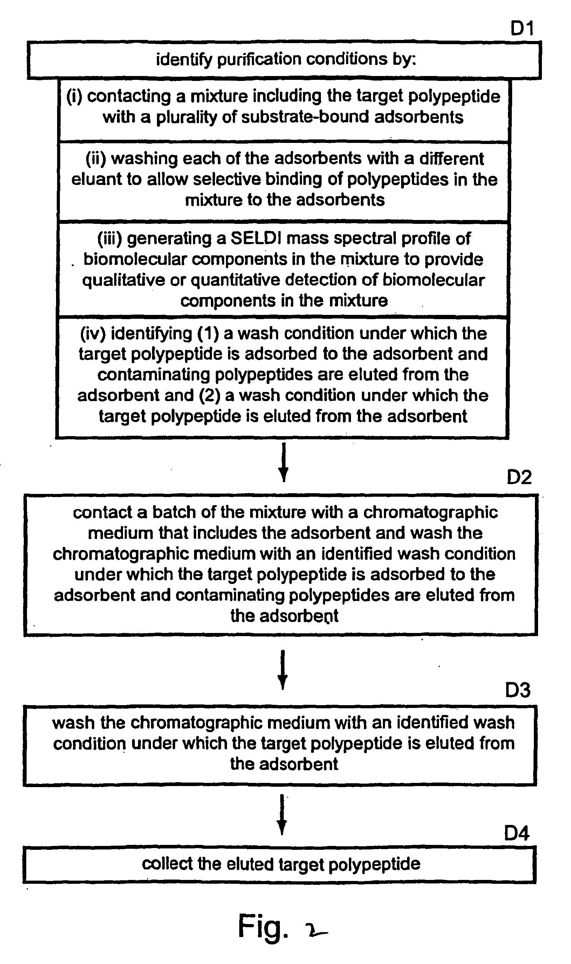

Figure 2 is a flow chart that schematically shows steps involved in an embodiment of a method of purifying a target polypeptide, which is optionally used to optimize a purification process for larger scale purifications. As shown, **D1** includes identifying purification conditions by: (i) contacting a mixture including the target polypeptide with a plurality of substrate-bound adsorbents (*e*.*g*., chromatographic adsorbents, biospecific adsorbents, or the like, *e*.*g*., in the form of a probe), (ii) washing each of the adsorbents with a different eluant to allow selective binding of polypeptides in the mixture to the adsorbents, (iii) generating a SELDI mass spectral profile of biomolecular components in the mixture to provide qualitative or quantitative detection of biomolecular components in the mixture, and (iv) identifying (1) a wash condition under which the target polypeptide is adsorbed to the adsorbent and contaminating polypeptides are eluted from the adsorbent and (2) a wash condition under which the target polypeptide is eluted from the adsorbent. Adsorbents and other aspects of SELDI mass spectral profiles are described in greater detail below. In **D2,** the method includes contacting a batch (*e*.*g*., at least about 1000 liters, *etc*.) of the mixture with a chromatographic medium that includes the adsorbent and washing the chromatographic medium with an identified wash condition under which the target polypeptide is adsorbed to the adsorbent and contaminating polypeptides are eluted from the adsorbent. In **D3,** the method further includes washing the chromatographic medium with an identified wash condition under which the target polypeptide is eluted from the adsorbent. The method also includes D4, collecting the eluted target polypeptide. The wash conditions typically include different parameters selected from one or more of, *e*.*g*., a salt concentration, a detergent concentration, a pH, a buffering capacity, an ionic strength, a water structure characteristic, a detergent type, a hydrophobicity, a dielectric constant, a concentration of at least one solvent (*e*.*g*., an organic solvent, *etc*.), a concentration of at least one solute, or the like.

### IV. BIOMOLECULE FRACTIONATION

While a sample comprising the target protein can be analyzed directly, in certain embodiments, the methods include fractionating biomolecules in an initial sample by one or a combination of fractionation techniques described herein or otherwise known in the art to be useful for separating biomolecules to collect a sample fraction that includes the target protein prior to mass profiling. Fractionation is typically utilized to decrease the complexity of analytes in the sample to assist detection and characterization of target proteins or impurities. Moreover, fractionation protocols can provide additional information regarding physical and chemical characteristics of biomolecular components in a sample. For example, if a sample is fractionated using an anion-exchange spin column, and if a target protein is eluted at a certain pH, this elution characteristic provides information regarding binding properties of the target protein. In another example, a sample can be fractionated to remove proteins or other molecules in the sample that are present in a high quantity and/or which would otherwise interfere with the detection of a particular target protein or a trace impurity.

Suitable sample fractionation protocols will be apparent to one of skill in the art. Exemplary fractionation techniques optionally utilized with the methods described herein include those based on size, such as size exclusion chromatography, gel electrophoresis, membrane dialysis, filtration, centrifugation (*e*.*g*., ultra-centrifugation), or the like. Separations are also optionally based on charges carried by analytes (*e*.*g*., as with anion or cation exchange chromatography), on analyte hydrophobicity (*e*.*g*., as with C₁-C₁₈ resins), on analyte affinity (*e*.*g*., as with immunoaffinity, immobilized metals, or dyes), or the like. In preferred embodiments, fractionation is effected using high performance liquid chromatography (HPLC). Other methods of fractionation include, *e*.*g*., crystallization and precipitation. Many of these fractionation techniques are described further in, *e*.*g*., Walker (Ed.) Basic Protein and Peptide Protocols: Methods in Molecular Biology, Vol. 32, The Humana Press (1994), Fallon et al. (Eds.) Applications of HPLC in Biochemistry: Laboratory Techniques in Biochemistry and Molecular Biology, Elsevier Science Publishers (1987), Matejtschuk (Ed.) Affinity Separations: A Practical Approach, IRL Press (1997), Scouten, Affinity Chromatography: Bioselective Adsorption on Inert Matrices, John Wiley & Sons (1981), Hydrophobic Interaction Chromatography: Principles and Methods, Pharmacia (1993), Brown, Advances in Chromatography, Marcel Dekker, Inc. (1998), Lough and Wainer (Eds.), High Performance Liquid Chromatography: Fundamental Principles and Practice, Blackie Academic and Professional (1996), Mant and Hodges (Eds.), High Performance Liquid Chromatography of Peptides and Proteins: Separation, Analysis and Conformation, CRC Press (1991), Weiss, Ion Chromatography, 2nd ed., VCH (1995), Ion-Exchange Chromatography: Principles and Methods, Pharmacia (1991), Smith, The Practice of Ion Chromatography, Krieger Publishing Company (1990), Bidlingmeyer, Practical HPLC Methodology and Applications, John Wiley & Sons, Inc. (1992), and Rickwood et al., Centrifugation: Essential Data Series, Cold Spring Harbor Laboratory (1994). Certain of these techniques are illustrated further below.

### A. SIZE EXCLUSION CHROMATOGRAPHY

In one embodiment, a sample can be fractionated according to the size of, *e*.*g*., proteins in a sample using size exclusion chromatography. For a biological sample in which the amount of sample available is small, preferably a size selection spin column is used. For example, K-30 spin column (Ciphergen Biosystems, Inc.) can be used. In general, the first fraction that is eluted from the column ("fraction 1") has the highest percentage of high molecular weight proteins; fraction 2 has a lower percentage of high molecular weight proteins; fraction 3 has even a lower percentage of high molecular weight proteins; fraction 4 has the lowest amount of large proteins; and so on. Each fraction is optionally then analyzed by gas phase ion spectrometry for the detection of particular proteins according to the methods described herein, such as the methods which detect the presence of a target polypeptide during production, the methods in which trace impurities are detected, *etc*. Examples that further illustrate the use of size exclusion chromatography are provided below.

### B. SEPARATION OF BIOMOLECULES BY GEL ELECTROPHORESIS

In another embodiment, biomolecules (*e*.*g*., proteins, nucleic acids, *etc*.) in a sample can be separated by high-resolution electrophoresis, *e*.*g*., one- or two-dimensional gel electrophoresis, Northern blotting, or the like. A fraction suspected of containing a target protein can be isolated and further analyzed by gas phase ion spectrometry as described herein. Preferably, two-dimensional gel electrophoresis is used to generate two-dimensional array of spots of biomolecules, including one or more target proteins. *See, e*.*g*., Jungblut and Thiede, Mass Spectr. Rev. 16:145-162 (1997).

Two-dimensional gel electrophoresis is optionally performed using methods known in the art. *See, e*.*g*., Deutscher ed., Methods In Enzymology vol. 182. Typically, biomolecules in a sample are separated by, *e*.*g*., isoelectric focusing, during which biomolecules in a sample are separated in a pH gradient until they reach a spot where their net charge is zero (*i*.*e*., their isoelectric point). This first separation step results in a one-dimensional array of biomolecules. The biomolecules in the one dimensional array are further separated using a technique generally distinct from that used in the first separation step. For example, in a second dimension, biomolecules separated by isoelectric focusing are further separated using a polyacrylamide gel, such as polyacrylamide gel electrophoresis in the presence of sodium dodecyl sulfate (SDS-PAGE). SDS-PAGE gel allows further separation based on molecular masses of biomolecules. Typically, two-dimensional gel electrophoresis can separate chemically different biomolecules in the molecular mass range from of from about 1000 to about 200,000 Da within complex mixtures.

Biomolecules in the two-dimensional array are optionally detected using any suitable method known in the art. For example, biomolecules in a gel can be labeled or stained (*e*.*g*., by Coomassie blue, silver staining, fluorescent tagging, radioactive labeling, or the like). If gel electrophoresis generates spots that correspond to the molecular weight of one or more target proteins, the spot can be is further analyzed by gas phase ion spectrometry according to the methods of the invention. For example, spots can be excised from the gel and proteins in the selected spot can be cleaved or otherwise fragmented into smaller peptide fragments using, *e*.*g*., cleaving reagents, such as proteases (*e*.*g*., trypsin), prior to gas phase ion spectrometeric analysis. Alternatively, the gel containing biomolecules can be transferred to an inert membrane by applying an electric field. Then, a spot on the membrane that approximately corresponds to the molecular weight of a marker can be analyzed according to the methods described herein. In gas phase ion spectrometry, the spots can be analyzed using any suitable technique, such as MALDI or surface enhanced laser desorption/ionization (*e*.*g*., using ProteinChip^{®} array) as described in detail below.

### C. HIGH PERFORMANCE LIQUID CHROMATOGRAPHY

In yet another embodiment, high performance liquid chromatography (HPLC) can be used to separate a mixture of biomolecules in a sample based on their different physical properties, such as polarity, charge, size, or the like. HPLC instruments typically consist of a mobile phase reservoir, a pump, an injector, a separation column, and a detector. Biomolecules in a sample are separated by injecting an aliquot of the sample onto the column. Different biomolecules in the mixture pass through the column at different rates due to differences in their partitioning behavior between the mobile liquid phase and the stationary phase. A fraction that corresponds to the molecular weight and/or physical properties of, *e*.*g*., one or more target proteins can be collected. The fraction can then be analyzed by gas phase ion spectrometry according to the methods described herein to detect the target proteins. For example, the spots can be analyzed using either MALDI or SELDI (*e*.*g*., using a ProteinChip^{®} array) as described in detail below.

### V BIOMOLECULE FRAGMENTATION

Prior to profiling biomolecule masses in a sample by gas phase ion spectroscopy, proteins in the samples of the invention are optionally fragmented or digested. This approach is particularly useful when components (*e*.*g*., protein impurities, *etc*.) of, *e*.*g*., a cell culture medium are to be identified. Fragmentation is optionally effected using any technique that produces peptide fragments from proteins in a sample. Many of these techniques are generally known in the art. For example, proteins are optionally fragmented enzymatically, chemically, or physically. In certain embodiments of the invention, target proteins and/or peptide fragments resulting from fragmentation are optionally modified to improve resolution upon detection. In other embodiments, the fragmentation of biomolecular components of a sample can be performed "on chip" in a SELDI environment by placing an aliquot of the sample on an adsorbent spot and adding, *e*.*g*., the proteolytic agent to the material on the spot. Additional details relating to the identification of biomolecules via fragmentation are described in, *e*.*g*., USSN 60/277,677 entitled "HIGH ACCURACY PROTEIN IDENTIFICATION," filed on March 20, 2001 by Thang.

### VI. BIOMOLECULE PROFILING

The invention includes profiling masses of components from purification processes, *e*.*g*., to qualitatively and/or quantitatively detect target polypeptides and/or impurities. In preferred embodiments, detection is via SELDI mass spectral profiles, which have significantly higher sensitivity levels than many other methods of detection. A review of SELDI and retentate chromatography is provided below.

### A. SELDI AND MALDI

SELDI differs from MALDI in the participation of the sample presenting surface in the desorption/ionization process. In MALDI, the sample presenting surface plays no role in this process - the analytes detected reflect those mixed with and trapped within the matrix material. In SELDI, the sample presenting surface comprises adsorbent molecules that exhibit some level of affinity for certain classes of analyte molecules. Thus, after application of energy absorbing molecules (*e*.*g*., "matrix") to the surface and impingement by an energy source, the specific analyte molecules detected depend, in part, upon the interaction between the adsorbent and the analyte molecules. Thus, different populations of molecules are detected when performing SELDI and MALDI.

Three different versions of SELDI are described here: "Retentate Chromatography," "No-wash SELDI" and "Concentration SELDI."

### 1. Retentate Chromatography

Retentate chromatography generally proceeds as follows. A liquid sample comprising bioorganic analytes, such as an aliquot of a cell culture supernatant is applied to a sample presenting surface which comprises an adsorbent, *e*.*g*., a spot on the surface of a probe or biochip. The adsorbent possesses various levels of affinity for classes of molecular analytes based on chemical characteristics. For example, a hydrophilic adsorbent has affinity for hydrophilic biomolecules. The sample is allowed to reach binding equilibrium with the adsorbent. In reaching binding equilibrium, the analytes bind to the adsorbent or remain in solution based on their level of attraction to the adsorbent.

The particular binding equilibrium achieved by a class of molecules is, of course, mediated by the binding constant of those molecules for the adsorbent. For example, the smaller the binding constant, the tighter the binding between the molecule and the adsorbent, and the more likely the molecule is to be bound to the adsorbent than to be in solution. Molecules that are non-attracted or repelled by the adsorbent are likely to be free in solution, with few, if any, being bound to the adsorbent.

After allowing molecules to bind to the adsorbent, the liquid and unbound molecules are removed from the spot, *e*.*g*., by pipetting. What is left on the spot are molecules bound to the adsorbent and probably some unbound molecules not completely removed with the liquid. Thus, most of the unbound molecules are removed with the removal of the liquid.

Then, a wash solution is applied to the spot. Generally, the wash solution has a different elution characteristic than the liquid in which the sample was applied. For example, the wash solution may have a different pH or salt concentration than that of the original sample. In the wash step, the analytes may reach a new equilibrium between being bound and remaining in solution. For example, if the stringency of the wash is greater than the stringency of the liquid in which the sample was applied, weakly bound molecules may be released into solution. This wash solution is now removed from the spot, taking with it unbound molecules. This includes both biomolecular analytes as well as inorganic molecules such as salts. Thus, the wash can function as a desalting step, particularly if the wash solution has similar characteristics to the solution in which the sample was applied.

After the wash step, the population of analyte molecules on the surface is significantly different from that of the population in the original sample. In particular, compared with molecules in the original sample, the ratio of molecules remaining on the adsorbent is heavily skewed toward those with particular affinity for the adsorbent, and molecules that have little or no affinity for the adsorbent have been removed by washing.

At this point, the analytes remaining on the surface are usually allowed to dry, although this step is not necessary. The analytes now exist as a layer on the spot.

Energy absorbing molecules (e.g., a cinnamic acid derivative, sinapinic acid, dihydroxybenzoic acid), sometimes called matrix, are applied to the probe surface to facilitate desorption/ionization. Usually, the energy absorbing molecules are applied to the spot and allowed to dry. However, in some embodiments, the energy absorbing molecules are applied to the surface of the probe before application of the sample. (One version of this embodiment is called "SEND." *See* U.S. patent 6,124,137 (Hutchens and Yip)). The analytes can now be examined by gas phase ion spectrometry, preferably laser desorption/ionization mass spectrometry; the interaction between the matrix and the surface layer of analytes at the interface between the two enabling desorption and ionization of biomolecular analytes at this interface.

### 2. No-wash SELDI

Another method, "No-wash SELDI," includes the following steps: A liquid sample comprising bioorganic analytes is applied to a sample presenting surface which comprises an adsorbent, *e*.*g*., a spot on the surface of a biochip. The sample is allowed to reach equilibrium with the adsorbent. After allowing molecules to bind to the adsorbent, the liquid is removed from the spot, e.g., by pipetting or the like. The bound molecules (and probably some unbound molecules) remain on the substrate and most of the unbound molecules are removed with the liquid. In this method, no wash solution is applied to the spot. Because excess sample is removed after reaching equilibrium, and without a wash step, the population of molecules on the adsorbent spot differs from the population of molecules in the applied sample and from the population remaining on the spot in retentate chromatography. As in retentate chromatography, the population on the adsorbent spot is richer in molecules having affinity for the adsorbent, compared with the originally applied sample. However, the population also differs from that remaining in retentate chromatography because un-bound, non-specifically bound or weakly bound molecules, which are washed away in retentate chromatography, remain on the sample presenting surface. This includes both biomolecular and inorganic species, such as salts.

At this point, the analytes remaining on the surface are usually allowed to dry, although this step is not necessary. Then, energy absorbing molecules (*e*.*g*., a cinnamic acid derivative, sinapinic acid and dihydroxybenzoic acid ) are applied to the spot and allowed to dry. Then, the analytes can be examined by gas phase ion spectrometry, preferably laser desorption/ionization mass spectrometry.

### 3. Concentration SELDI

In another method, referred to as "Concentration SELDI," the steps proceed as follows. A liquid sample comprising bioorganic analytes is applied to a sample presenting surface which comprises an adsorbent, *e*.*g*., a spot on the surface of a biochip. The analytes in the sample are now concentrated on the adsorbent surface. Concentration proceeds by reducing the volume of the sample (*e*.*g*., by evaporation) so that the amount of analyte per unit volume increases. In contrast to no-wash SELDI or retentate chromatography, sample liquid and unbound analytes are not removed together from the adsorbent surface. The analytes in the sample are preferably concentrated essentially to dryness. However, concentration can proceed at least 2-fold, at least 10-fold, at least 100-fold, or at least 1000 fold before application of energy absorbing molecules. Because the volume of the sample decreases steadily, the analytes never reach a stable binding equilibrium in solution. By concentrating the analytes on the adsorbent, all the analytes in the sample remain on the surface, regardless of their attraction to the adsorbent. (Certain volatile analytes may be lost in an evaporation process.) Thus, there is both specific binding (*i*.*e*., adsorbent mediated) and non-specific binding of analytes to the adsorbent surface. Then, energy absorbing molecules are applied to the spot and allowed to dry. Then, the analytes can be examined by gas phase ion spectrometry, preferably laser desorption/ionization mass spectrometry.

In this case, while the population of analytes on the surface of the chip reflects the population of analytes in the applied sample, a fraction of the analytes remain bound to the chip surface even after the application of an energy absorbing material. Thus, the analyte fraction incorporated into the energy absorbing material represents the fraction of analytes which have low binding affinity for the adsorbent surface under the conditions present when the solution of energy absorbing material is deposited on the adsorbent surface. This contrasts with MALDI, in which the analyte sample is mixed directly with matrix material. The result is that signal strength from an analyte in each case is different, and signals from certain molecules, which are not detectable or distinguishable in MALDI can be detected in concentration SELDI. Thus, concentration SELDI can provide a more sensitive assay for the presence of bioorganic molecules in a sample than MALDI.

### B. CONTACTING A BIOMOLECULE SAMPLE WITH A SUBSTRATE FOR GAS PHASE ION SPECTROMETRIC ANALYSIS

### 1. Analysis of an unfractionated samples

In certain embodiments, samples are analyzed without being fractionated prior to examination by gas phase ion spectrometry, e.g., MALDI or SELDI. For example, a sample of a cell culture supernatant is optionally analyzed directly from a cell culture medium to assess the presence of a secreted target polypeptide. Another option includes, analyzing samples taken from various stages of a purification process in the absence of fractionation prior to mass profiling.

In MALDI, the sample is usually mixed with an appropriate matrix, placed on the surface of a probe and examined by laser desorption/ionization. The technique of MALDI is well known in the art. *See, e*.*g*., U.S. patent 5,045,694 (Beavis et al.), U.S. patent 5,202,561 (Gleissmann et al.), and U.S. Patent 6,111,251 (Hillenkamp). However, MALDI frequently does not provide results as good as analysis by SELDI.

In SELDI, the first aliquot is contacted with a solid phase-bound (*e*.*g*., substrate-bound) adsorbent. A substrate is typically a probe (*e*.*g*., a biochip) that is removably insertable into a gas phase ion spectrometer. In SELDI-based applications of the present invention, a probe generally includes a substrate with at least one surface feature having at least one adsorbent, bound to the substrate, that is capable of capturing, *e*.*g*., one or more peptide fragments from target proteins. A preferred adsorbent for this application is a normal phase or hydrophilic adsorbent, *e*.*g*., silicon oxide. Probes are described in greater detail below.

Alternatively, the substrate can be a solid phase, such as a polymeric, paramagnetic, latex, or glass bead or resin comprising, *e*.*g*., a functional group or adsorbent for binding peptide fragments. After capture of the analyte, the solid phase is placed on a probe that is removably insertable into a gas phase ion spectrometer.

A sample is contacted with a probe comprising an adsorbent, by any suitable manner, such as bathing, soaking, dipping, spraying, washing over, pipetting, *etc*. Generally, a volume of a sample aliquot containing from a few attomoles to 100 picomoles of peptide fragments in about 1 µl to 500 µl of a solvent is sufficient for binding to an adsorbent. The sample aliquot can contact the probe substrate comprising an adsorbent for a period of time sufficient to allow peptide fragments to bind to the adsorbent. Typically, the sample aliquot and a substrate comprising an adsorbent are contacted for a period of between about 30 seconds and about 12 hours, and preferably, between about 30 seconds and about 15 minutes. Furthermore, the sample aliquot is generally contacted to the probe substrate under ambient temperature and pressure conditions. For some sample aliquots, however, modified temperature (typically 4°C through 37°C) and pressure conditions can be desirable, which conditions are determinable by those skilled in the art.

The sample is allowed to dry on the spot, or, after a suitable time, the excess sample is removed from the spot. Thereafter, peptide fragments in the first aliquot are desorbed and ionized from the probe and detected using gas phase ion spectrometry to provide a first set of peptide fragment mass data. The first set of peptide fragment mass data generally provides a profile of all or most peptide fragments present in the sample aliquot.

### 2. Analysis of the fractionated samples

Samples are optionally analyzed, according to the methods of the present invention, after fractionation of the samples. Example which illustrate the use of sample fractionation according the methods described herein are provided below. Fractionation of a sample aliquot typically increases the total information content about biomolecules present in the particular sample. For example, fractionation may result in the detection of trace impurities that would otherwise be undetectable, or not accurately detected, in an unfractionated sample by eliminating signals attributable to more abundant biomolecular components that would otherwise suppress the signals of less abundant components. Further, biomolecules remaining in the sample after fractionation are typically detected with improved mass accuracy as a result of an increased signal:noise ratio. The use of information about sample components from fractionated samples as well as unfractionated samples generally leads to a higher confidence level that a given target protein or impurity has been accurately detected.

The fractionation steps that generate sample fractions can be performed by any of the fractionation methods described above. For example, prior to spectrometrically profiling biomolecular component masses in a particular sample, biomolecules in the sample are separated into fractions using, *e*.*g*., HPLC. In a preferred embodiment, the fractionation and analysis is performed by SELDI/retentate chromatography, which is now described in more detail.

In one embodiment, these fractionated samples are analyzed by typical MALDI methods, such as those described above, in which the sample is applied to a probe surface that is not actively involved in the desorption/ionization of the analyte from the probe surface.

However, in a preferred embodiment, fractionating and analyzing the sample is performed by retentate chromatography. Retentate chromatography involves directly contacting an aliquot with an adsorbent bound to a surface of a probe in which the adsorbent captures one or more biomolecular components, such as a target protein and/or an impurity. This embodiment also includes removing non-captured material from the probe, *e*.*g*., by one or more washes prior to gas phase ion spectrometric analysis. Optionally, the sample is indirectly contacted with a probe surface after being contacted with a support-bound adsorbent that captures one or more sample components. Non-captured materials are optionally removed (*e*.*g*., by one or more washes) before or after the support-bound adsorbent is contacted with the probe surface.

Washing to remove non-captured materials can be accomplished by, *e*.*g*., bathing, soaking, dipping, rinsing, spraying, or washing the substrate surface, or a support-bound adsorbent, following exposure to the sample with an eluant. A microfluidics process is preferably used when an eluant is introduced to small spots (*e*.*g*., surface features) of adsorbents on the probe. Typically, the eluant can be at a temperature of between 0°C and 100°C, preferably between 4°C and 37°C. Any suitable eluant (*e*.*g*., organic or aqueous) can be used to wash the substrate surface. For example, each of the one or more washes optionally includes an identical or a different elution condition relative to at least one preceding wash. Elution conditions typically differ according to, *e*.*g*., pH, buffering capacity, ionic strength, a water structure characteristic, detergent type, detergent strength, hydrophobicity, dielectric constant, concentration of at least one solute, or the like. Preferably, an aqueous solution is used. Exemplary aqueous solutions include a HEPES buffer, a Tris buffer, or a phosphate buffered saline, *etc*. To increase the wash stringency of the buffers, additives can be incorporated into the buffers. These include, but are not limited to, ionic interaction modifiers (both ionic strength and pH), water structure modifiers, hydrophobic interaction modifiers, chaotropic reagents, affinity interaction displacers. Specific examples of these additives can be found in, *e*.*g*., PCT publication WO98/59360 (Hutchens and Yip). The selection of a particular eluant or eluant additives is dependent on other experimental conditions (*e*.*g*., types of adsorbents used or peptide fragments to be detected), and can be determined by those of skill in the art.

An option to read molecules with very large masses, such as IgM antibodies, is to treat them in reducing conditions so that they produce smaller fragments. This results not from digestion, but rather from a dissociation of disulfur bonds (when present). In the case of antibodies molecules, this produces heavy and light chains that are both smaller than the whole antibody and more easily detected by mass spectrometry.

Prior to desorption and ionization of biomolecules from a probe surface according to any of the methods described herein, a energy absorbing molecules or a matrix material is typically applied to a given sample on the substrate surface, usually after allowing the sample to dry. The energy absorbing molecules can assist absorption of energy from an energy source from a gas phase ion spectrometer, and can assist desorption of biomolecular components from the probe surface. Exemplary energy absorbing molecules include cinnamic acid derivatives, sinapinic acid ("SPA"), cyano hydroxy cinnamic acid ("CHCA") and dihydroxybenzoic acid. Other suitable energy absorbing molecules are known to those skilled in the art. *See, e*.*g*., U.S. Patent 5,719,060 (Hutchens & Yip) for additional description of energy absorbing molecules.

The energy absorbing molecule and the biomolecular components in a given sample fraction can be contacted in any suitable manner. For example, an energy absorbing molecule is optionally mixed with a sample fraction and the mixture is placed on the substrate surface, as in a traditional MALDI process. In another example, an energy absorbing molecule can be placed on the substrate surface prior to contacting the substrate surface with a sample fraction. In another example, a sample fraction can be placed on the substrate surface prior to contacting the substrate surface with an energy absorbing molecule. Then, the biomolecular components in the sample fraction can be desorbed, ionized and detected as described in detail below.

Optionally, multiple fractions of a given sample are analyzed in parallel. Additional options include analyzing unfractionated and fractionated samples in parallel. However, in other embodiments of the invention, these analyses can be performed in series. For example, a unfractionated sample aliquot can be placed on a spot and allowed to equilibrate. Then the remaining liquid in the sample can be transferred to an adsorbent spot for fractionation by retentate chromatography.

### 3. Probes

A probe (*e*.*g*., a biochip) is optionally formed in any suitable shape (*e*.*g*., a square, a rectangle, a circle, or the like) as long as it is adapted for use with a gas phase ion spectrometer (*e*.*g*., removably insertable into a gas phase ion spectrometer). For example, the probe can be in the form of a strip, a plate, or a dish with a series of wells at predetermined addressable locations or have other surfaces features. The probe is also optionally shaped for use with inlet systems and detectors of a gas phase ion spectrometer. For example, the probe can be adapted for mounting in a horizontally, vertically and/or rotationally translatable carriage that horizontally, vertically and/or rotationally moves the probe to a successive position without requiring repositioning of the probe by hand.

In certain embodiments, the probe substrate surface can be conditioned to bind analytes. For example, in one embodiment, the surface of the probe substrate can be conditioned (*e*.*g*., chemically or mechanically such as roughening) to place adsorbents on the surface. The adsorbent comprises functional groups for binding with an analyte. In some embodiments, the substrate material itself can also contribute to adsorbent properties and may be considered part of an "adsorbent."

Adsorbents can be placed on the probe substrate in continuous or discontinuous patterns. If continuous, one or more adsorbents can be placed on the substrate surface. If multiple types of adsorbents are used, the substrate surface can be coated such that one or more binding characteristics vary in a one- or two-dimensional gradient. If discontinuous, plural adsorbents can be placed in predetermined addressable locations or surface features (*e*.*g*., addressable by a laser beam of a mass spectrometer) on the substrate surface. The surface features of probes or biochips include various embodiments. For example, a biochip optionally includes a plurality of surface features arranged in, *e*.*g*., a line, an orthogonal array, a circle, or an n-sided polygon, wherein n is three or greater. The plurality of surface features typically includes a logical or spatial array. Optionally, each of the plurality of surface features comprises identical or different adsorbents, or one or more combinations thereof. For example, at least two of the plurality of surface features optionally includes identical or different adsorbents, or one or more combinations thereof. Suitable adsorbents are described in greater detail below.

The probe substrate can be made of any suitable material. Probe substrates are preferably made of materials that are capable of supporting adsorbents. For example, the probe substrate material can include, but is not limited to, insulating materials (*e*.*g*., plastic, ceramic, glass, or the like), a magnetic material, semiconducting materials (*e*.*g*., silicon wafers), or electrically conducting materials (*e*.*g*., metals, such as nickel, brass, steel, aluminum, gold, metalloids, alloys or electrically conductive polymers), polymers, organic polymers, conductive polymers, biopolymers, native biopolymers, metal coated with organic polymers, synthetic polymers, composite materials or any combinations thereof. The probe substrate material is also optionally solid or porous.

Probes are optionally produced using any suitable method depending on the selection of substrate materials and/or adsorbents. For example, the surface of a metal substrate can be coated with a material that allows derivatization of the metal surface. More specifically, a metal surface can be coated with silicon oxide, titanium oxide, or gold. Then, the surface can be derivatized with a bifunctional linker, one end of which can covalently bind with a functional group on the surface and the other end of which can be further derivatized with groups that function as an adsorbent. In another example, a porous silicon surface generated from crystalline silicon can be chemically modified to include adsorbents for binding analytes. In yet another example, adsorbents with a hydrogel backbone can be formed directly on the substrate surface by *in situ* polymerizing a monomer solution that includes, *e*.*g*., substituted acrylamide monomers, substituted acrylate monomers, or derivatives thereof comprising a selected functional group as an adsorbent. Probes suitable for use in the invention are described in, e.g., U.S. Patent No. 5,617,060 (Hutchens and Yip) and WO 98/59360 (Hutchens and Yip).

### 4. Adsorbents

In some embodiments, the complexity of a sample can be further reduced using a substrate that comprises adsorbents capable of binding one or more sample components (e.g., a target protein, an impurity, *etc*.). A plurality of adsorbents are optionally utilized in the methods of this invention. Different adsorbents can exhibit grossly different binding characteristics, somewhat different binding characteristics, or subtly different binding characteristics. For example, adsorbents need not be biospecific (*e*.*g*., biomolecular interaction adsorbents, such as antibodies that bind specific target polypeptides) as long as the adsorbents have binding characteristics suitable for binding a biomolecular component with a particular characteristic from the sample. For example, adsorbents optionally include chromatographic adsorbents, such as a hydrophobic interaction adsorbent or group, a hydrophilic interaction adsorbent or group, a cationic adsorbent or group, an anionic adsorbent or group, a metal-chelating adsorbent or group (*e*.*g*., nickel, cobalt, *etc*.), lectin, heparin, or any combination thereof. In other embodiments, adsorbents include biomolecular interaction adsorbents, such as affinity adsorbents, polypeptides, enzymes, receptors, antibodies, or the like. For example, in certain embodiments, a biomolecular interaction adsorbent includes a monoclonal antibody that captures specific target proteins.

Adsorbents which exhibit grossly different binding characteristics typically differ in their bases of attraction or mode of interaction. The basis of attraction is generally a function of chemical or biological molecular recognition. Bases for attraction between an adsorbent and an analyte, such as a polypeptide include, *e*.*g*., (1) a salt-promoted interaction, *e*.*g*., hydrophobic interactions, thiophilic interactions, and immobilized dye interactions, (2) hydrogen bonding and/or van der Waals force interactions and charge transfer interactions, *e*.*g*., hydrophilic interactions, (3) electrostatic interactions, such as an ionic charge interaction, particularly positive or negative ionic charge interactions, (4) the ability of the analyte to form coordinate covalent bonds (*i*.*e*., coordination complex formation) with a metal ion on the adsorbent, or (5) combinations of two or more of the foregoing modes of interaction. That is, the adsorbent can exhibit two or more bases of attraction, and thus be known as a "mixed functionality" adsorbent.

### a) Salt-Promoted Interaction Adsorbents

Adsorbents that are useful for observing salt-promoted interactions include hydrophobic interaction adsorbents. Examples of hydrophobic interaction adsorbents include matrices having aliphatic hydrocarbons (*e*.*g*., C₁-C₁₈ aliphatic hydrocarbons) and matrices having aromatic hydrocarbon functional groups (*e*.*g*., phenyl groups or heterocycles). Another adsorbent useful for observing salt-promoted interactions includes thiophilic interaction adsorbents, such as T-GEL® which is one type of thiophilic adsorbent commercially available from Pierce, Rockford, Illinois. A third adsorbent which involves salt-promoted ionic interactions and also hydrophobic interactions includes immobilized dye interaction adsorbents.

### (i) Reverse Phase Adsorbent - Aliphatic Hydrocarbon

One useful reverse phase adsorbent is a hydrophobic adsorbent which is present on an H4 ProteinChip® array, available from Ciphergen Biosystems, Inc. (Fremont, CA). The hydrophobic H4 chip comprises aliphatic hydrocarbon chains immobilized on top of silicon oxide (SiO₂) as the adsorbent on the substrate surface.

### b) Hydrophilic Interaction Adsorbents

Adsorbents which are useful for observing hydrogen bonding and/or van der Waals forces on the basis of hydrophilic interactions include surfaces comprising normal phase adsorbents such as silicon oxide (SiO₂), titanium oxide, aluminum oxide and zirconium oxide. The normal phase or silicon-oxide surface acts as a functional group. In addition, adsorbents comprising surfaces modified with hydrophilic polymers such as polyethylene glycol, dextran, agarose, or cellulose can also function as hydrophilic interaction adsorbents. Most proteins will bind hydrophilic interaction adsorbents because of a group or combination of amino acid residues (*i*.*e*., hydrophilic amino acid residues) that bind through hydrophilic interactions involving hydrogen bonding or van der Waals forces.

### (i) Normal Phase Adsorbent - Silicon Oxide

One useful hydrophilic adsorbent is presented on a Normal Phase (NP) ProteinChip® array, available from Ciphergen Biosystems, Inc. (Fremont, CA). The normal phase chip comprises silicon oxide as the adsorbent on the substrate surface. Silicon oxide can be applied to the surface by any of a number of well known methods. These methods include, for example, vapor deposition, *e*.*g*., sputter coating. A preferred thickness for such a probe is about 9000 Angstroms.

### c) Electrostatic Interaction Adsorbents

Adsorbents which are useful for observing electrostatic or ionic charge interactions include anion exchangers such as, for example, matrices of sulfate anions (*i*.*e*., SO₃⁻) and matrices of carboxylate anions (*i*.*e*., COO⁻) or phosphate anions (*i*.*e*., PO₄⁻). Matrices having sulfate anions have permanent negative charges. However, matrices having carboxylate anions have a negative charge only at a pH above their pKa. At a pH below the pKa, the matrices exhibit a substantially neutral charge. Suitable anionic adsorbents also include anionic adsorbents which are matrices having a combination of sulfate and carboxylate anions and phosphate anions.

Other adsorbents which are useful for observing electrostatic or ionic charge interactions include cation exchangers. Specific examples of cationic adsorbents include matrices of secondary, tertiary or quaternary amines. Quaternary amines are permanently positively charged. However, secondary and tertiary amines have charges that are pH dependent. At a pH below the pKa, secondary and tertiary amines are positively charged, and at a pH above their pKa, they are negatively charged. Suitable cationic adsorbents also include cationic adsorbents which are matrices having combinations of different secondary, tertiary, and quaternary amines.

In the case of ionic interaction adsorbents (both anionic and cationic) it is often desirable to use a mixed mode ionic adsorbent containing both anions and cations. Such adsorbents provide a continuous buffering capacity as a function of pH. Other adsorbents that are useful for observing electrostatic interactions include, *e*.*g*., dipole-dipole interaction adsorbents in which the interactions are electrostatic but no formal charge donor or acceptor is involved.

### (i) Anionic Adsorbent

One useful adsorbent is an anionic adsorbent as presented on the SAX1 or SAX2 ProteinChip® array made by Ciphergen Biosystems, Inc. (Fremont, CA). The SAX1 protein chips are fabricated from SiO₂ coated aluminum substrates. In the process, a suspension of quaternary ammonium polystryenemicrospheres in distilled water is deposited onto the surface of the chip (1 mL/spot, two times). After air drying (room temperature, 5 minutes), the chip is rinsed with deionized water and air dried again (room temperature, 5 minutes).

### (ii) Cationic Adsorbent

A useful adsorbent is an cationic adsorbent as presented on the SCX1 or SCX2 ProteinChip® array made by Ciphergen Biosystems, Inc. (Fremont, CA). The SCX1 protein chips are fabricated from SiO₂ coated aluminum substrates. In the process, a suspension of sulfonate polystyrene microspheres in distilled water is deposited onto the surface of the chip (1 mL/spot, two times). After air drying (room temperature, 5 minutes), the chip is rinsed with deionized water and air dried again (room temperature, 5 minutes).

### d) Coordinate Covalent Interaction Adsorbents

Adsorbents which are useful for observing the ability to form coordinate covalent bonds with metal ions include matrices bearing, for example, divalent and trivalent metal ions. Matrices of immobilized metal ion chelators provide immobilized synthetic organic molecules that have one or more electron donor groups which form the basis of coordinate covalent interactions with transition metal ions. The primary electron donor groups functioning as immobilized metal ion chelators include oxygen, nitrogen, and sulfur. The metal ions are bound to the immobilized metal ion chelators resulting in a metal ion complex having some number of remaining sites for interaction with electron donor groups on the analyte. Suitable metal ions include in general transition metal ions such as copper, nickel, cobalt, zinc, iron, and other metal ions such as aluminum and calcium.

### (i) Nickel Chelate Adsorbents

Another useful adsorbent is a metal chelate adsorbent as presented on the IMAC3 (Immobilized Metal Affinity Capture, nitrilotriacetic acid on surface) ProteinChip® array, also available from Ciphergen Biosystems, Inc. (Fremont, CA). The chips are produced as follows: 5-Methacylamido-2-(N,N-biscarboxymethaylamino)pentanoic acid (7.5 wt%), Acryloyltri-(hydroxymethyl)methylamine (7.5 wt%), and N,N'-methylenebisacrylamide (0.4 wt%) are photo-polymerized using (-)riboflavin (0.02 wt%) as a photo-initiator. The monomer solution is deposited onto a rough etched, glass coated substrate (0.4 mL, twice) and irradiated for 5 minutes with a near UV exposure system (Hg short arc lamp, 20 mW/cm² at 365 nm). The surface is washed with a solution of sodium chloride (1 M) and then washed twice with deionized water.

The IMAC3 with Ni(II) is activated as follows. The surface is treated with a solution of NiSO₄ (50 mM, 10 mL/spot) and mixed on a high frequency mixer for 10 minutes. After removing the NiSO₄ solution, the treatment process is repeated. Finally, the surface is washed with a stream of deionized water (15 sec/chip).

### e) Enzyme-Active Site Interaction Adsorbents

Adsorbents which are useful for observing enzyme-active site binding interactions include proteases (such as trypsin), phosphatases, kinases, glycohydrolases and nucleases. The interaction is a sequence-specific interaction of the enzyme binding site on the analyte (typically a biopolymer) with the catalytic binding site on the enzyme.

### f) Reversible Covalent Interaction Adsorbents

Adsorbents which are useful for observing reversible covalent interactions include disulfide exchange interaction adsorbents. Disulfide exchange interaction adsorbents include adsorbents comprising immobilized sulfhydryl groups, *e*.*g*., mercaptoethanol, immobilized dithiothrietol or mercury-containing molecules able to react with sulfidryl grous. The interaction is based upon the formation of covalent disulfide bonds or mercury sulfur bonds between the adsorbent and solvent exposed cysteine residues or protein chemically modified so that to carry SH groups on the analyte. Such adsorbents bind proteins or peptides having cysteine residues and nucleic acids including bases modified to contain reduced sulfur compounds.

### g) Glycoprotein Interaction Adsorbents

Adsorbents which are useful for observing glycoprotein interactions include glycoprotein interaction adsorbents such as adsorbents having immobilize lectins (*i*.*e*., proteins bearing oligosaccharides) therein, an example of which is Conconavalin A, which is commercially available from, *e*.*g*., Sigma Chemical Company (St. Louis, MO). Such adsorbents function on the basis of the interaction involving molecular recognition of carbohydrate moieties on macromolecules.

### h) Biospecific Interaction Adsorbents

Adsorbents which are useful for observing biospecific interactions are generically termed "biospecific affinity adsorbents." Adsorption is considered biospecific if it is selective and the affinity (equilibrium dissociation constant, K_{d}) is at least 10⁻³ M to (*e*.*g*., 10⁻⁵ M, 10⁻⁷ M, 10⁻⁹ M, or the like). Examples of biospecific affinity adsorbents include any adsorbent which specifically interacts with and binds a particular biomolecule. Biospecific affinity adsorbents include for example, immobilized antibodies which bind to antigens, *e*.*g*., specific peptide fragments, immobilized receptors, or the like.

### i) Tag Interaction Adsorbents

Adsorbents which are useful for observing tagged molecules are referred to herein as "tag interaction adsorbents." These include, for example, chelating groups to interact with his-tagged proteins; biotin to interact with avidine/streptavidine tagged proteins; glutathione to interact with GST-tagged proteins; amylose to interact with MalE-tagged protein; and cellulose to interact with CBD-tagged proteins.

### VII. GAS PHASE ION SPECTROMETRY

In preferred embodiments, biomolecular components, such as target polypeptides and/or impurities in a sample aliquot are detected using gas phase ion spectrometry, and more preferably, using mass spectrometry. In one embodiment, matrix-assisted laser desorption/ionization ("MALDI") mass spectrometry is used, *e*.*g*., to profile biomolecule masses in a sample. In MALDI, the sample is typically quasi-purified to obtain a fraction that essentially consists of a target protein using, *e*.*g*., protein separation methods such as two-dimensional gel electrophoresis, HPLC, or the like. Biomolecule fractionation techniques are described in greater detail above. Additional details relating to MALDI are included in, *e*.*g*., Skoog et al., Principles of Instrumental Analysis, 5th Ed., Harcourt Brace & Co. (1998) and Siuzdak, Mass Spectrometry for Biotechnology, Academic Press (1996). Systems that include gas phase ion spectrometers are described further below.

In preferred embodiments, SELDI mass spectrometry is optionally used to desorb and ionize biomolecules from probe surfaces. SELDI uses a substrate comprising adsorbents to capture peptide fragments, which are then optionally directly desorbed and ionized from the substrate surface during mass spectrometry. Since the substrate surface in surface enhanced laser desorption/ionization captures sample components, a sample need not be quasi-purified as in MALDI. However, depending on the complexity of a sample and the type of adsorbents used, it is typically desirable to prepare a sample aliquot with reduced complexity by, *e*.*g*., removing non-captured materials prior to surface enhanced laser desorption/ionization analysis.

To illustrate, Figure 3 -schematically shows a surface enhanced laser desorption/ionization assay of an unfractionated sample that includes chromatographic adsorbent **506** on biochip **502.** Chromatographic adsorbents such as hydrophobic and hydrophilic interaction adsorbents are described further above. As additionally described above, biomolecular components **504** in the sample are not washed after being placed on chromatographic adsorbent **506** which is bound to surface feature **500.** Incident photon energy from laser **508** causes the desorption and ionization of biomolecular components **504,** which are then detected in a mass spectrometer to produce mass spectra **510.**

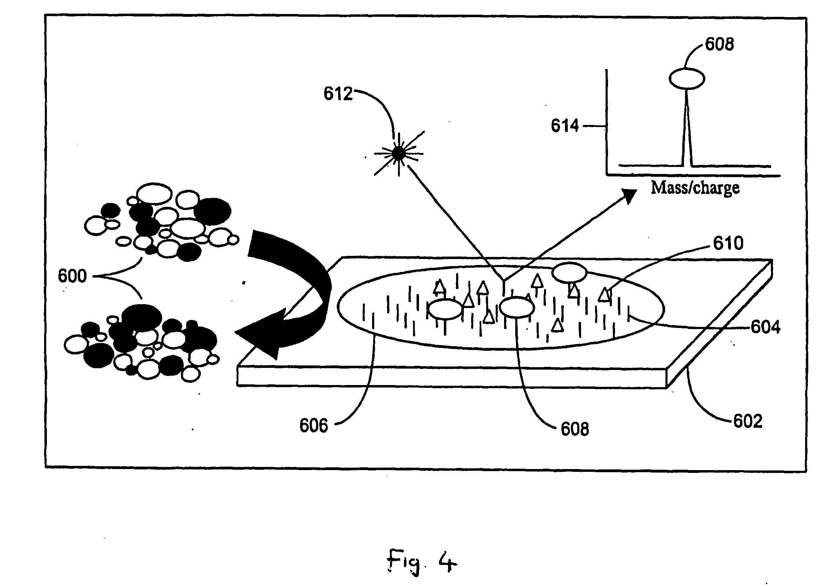

Figure 4 schematically illustrates a surface enhanced laser desorption/ionization assay of a sample, such as one taken from a cell culture supenatant that include secreted target proteins. As depicted, sample **600** is applied to biochip **602** which includes chromatographic adsorbent **604** bound to surface feature **606.** Components of sample **600** that are not bound to chromatographic adsorbent **604** are washed away (*e*.*g*., eluted or the like) from biochip **602** prior to mass analysis, as described above. Following capture and washing of target polypeptide **608** in sample **600,** energy absorbing molecules **610** (not shown in Figure 3) are added to biochip **602** to absorb energy from ionization source **612** (*i*.*e*., a laser) to aid desorption of target polypeptide **608** from the surface of biochip **602.** Mass spectrum **614** is produced by mass spectral analysis of desorbed/ionized target polypeptide **608.**

Optionally, any suitable gas phase ion spectrometer is used as long as it allows biomolecular components on the substrate to be resolved and detected. For example, in certain embodiments the gas phase ion spectrometer is a mass spectrometer. In a typical mass spectrometer, a probe comprising biomolecular components on its surface is introduced into an inlet system of the mass spectrometer. The biomolecular components are then desorbed by a desorption source such as a laser, fast atom bombardment, high energy plasma, electrospray ionization, thermospray ionization, liquid secondary ion MS, field desorption, *etc*. The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of biomolecular components or other substances will typically involve detection of signal intensity. This, in turn, can reflect the quantity and character of biomolecular components bound to the substrate. Any of the components of a mass spectrometer (*e*.*g*., a desorption source, a mass analyzer, a detector, *etc*.) can be combined with other suitable components described herein or others known in the art in embodiments of the invention.

In preferred aspects, a laser desorption time-of-flight mass spectrometer is used in embodiments of the invention. In laser desorption mass spectrometry, a substrate or a probe comprising biomolecular components is introduced into an inlet system. The materials are desorbed and ionized into the gas phase by incident laser energy from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of peptide fragments of specific mass-to-charge ratios.

In another embodiment, an ion mobility spectrometer is optionally used to detect biomolecular components. The principle of ion mobility spectrometry is based on different ion mobilities. Specifically, ions of a sample produced by ionization move at different rates, due to their difference in, *e*.*g*., mass, charge, or shape, through a tube under the influence of an electric field. The ions (typically in the form of a current) are registered at the detector which can then be used to identify a biomolecular component in a sample. One advantage of ion mobility spectrometry is that it can operate at atmospheric pressure.

In yet another embodiment, a total ion current measuring device is optionally used to detect and characterize biomolecular components. This device is optionally used when the substrate has only a single type of marker. When a single type of marker is on the substrate, the total current generated from the ionized marker reflects the quantity and other characteristics of the marker. The total ion current produced by the marker can then be compared to a control (*e*.*g*., a total ion current of a known compound). The quantity or other characteristics of the marker can then be determined.

In still another embodiment, quadrupole time-of-flight (Q-TOF) mass spectrometers, which are capable of tandem mass spectrometry, are optionally utilized to perform the methods described herein. These mass analyzer systems are readily coupled to laser desorption/ionization sources and are routinely used for protein and peptide analyses. Many Q-TOF mass spectrometers include mass ranges in excess of m/z 10000 and resolving powers of about 10000 full-width half maximum.

### VIII. DATA ANALYSIS

Data generated by desorption and detection of biomolecular components is optionally analyzed using any suitable method, *e*.*g*., to identify and/or quantify detected components. In one embodiment, data is analyzed with the use of a logic device, such as a programmable digital computer that is included, *e*.*g*., as part of a system. Systems are described further below. The computer generally includes a computer readable medium that stores logic instructions of the system software. Certain logic instructions are typically devoted to memory that includes the location of each feature on a probe, the identity of the adsorbent at that feature, the elution conditions used to wash the adsorbent, or the like. The computer also typically includes logic instructions that receives as input, data on the strength of the signal at various molecular masses received from a particular addressable location or surface feature on the probe, and instructions for entering data into a database. This data generally indicates the number and masses of components detected, including the strength of the signal generated by each component.

In certain embodiments, sets of biomolecular component mass data (*e*.*g*., first set, second set, *etc*.) are in a computer-readable form suitable for use in database queries. For example, a database query generally includes operating the programmable computer or other logic device and executing an algorithm that determines closeness-of-fit between the computer-readable data and database entries. The database entries typically correspond to masses of identified proteins or other compounds, which are correlated biomolecular component mass data to produce identity candidates for detected components in a particular sample. Essentially any protein or other biomolecule database is optionally queried with the biomolecular component mass data obtained using the methods and systems of the present invention. Many suitable databases are available and generally known in the art. In some embodiments, the algorithm includes an artificial intelligence algorithm or a heuristic learning algorithm. For example, the artificial intelligence algorithm optionally includes one or more of, *e*.*g*., a fuzzy logic instruction set, a cluster analysis instruction set, a neural network, a genetic algorithm, or the like.

Various software packages are currently available for querying databases, improving the speed of mass spectrometric protein identification processes, and otherwise integrating mass spectrometry into bioinformatics. For example, Mascot is a search engine that uses mass spectrometry data to identify proteins from primary sequence databases. *See, e*.*g*., Perkins et al. (1999) "Probability-based protein identification by searching sequence databases using mass spectrometry data," Electrophoresis 20(18):3551-3567. Another exemplary software package that is useful for performing the methods of the present invention includes ProFound, which performs rapid database searching combined with Bayesian statistics for protein identification. Profound is described further in, *e*.*g*., Zhang and Chait (2000) "ProFound-An expert system for protein identification using mass spectrometric peptide mapping information," Anal. Chem. 72:2482-8249, Zhang and Chait (1998) "ProFound-An expert system for protein identification," Proceedings of the 46th ASMS Conference on Mass Spectrometry and Allied Topics, Orlando, Florida, p.969, and Zhang and Chait (1995) "Protein identification by database searching: a Bayesian algorithm," Proceedings of the 43rd ASMS Conference on Mass Spectrometry and Allied Topics, Atlanta, Georgia, p. 643.

Data analysis also generally includes the steps of determining signal strength (*e*.*g*., height of peaks) of an analyte detected and removing "outliers" (data deviating from a predetermined statistical distribution). The observed peaks can be normalized, a process whereby the height of each peak relative to some reference is calculated. For example, a reference can be background noise generated by an instrument and chemicals (*e*.*g*., energy absorbing molecules) which is set as zero in the scale. Then the signal strength detected for each marker or other biomolecules can be displayed in the form of relative intensities in the scale desired (*e*.*g*., 100). Alternatively, a standard (*e*.*g*., bovine serum albumin) may be admitted with the sample so that a peak from the standard can be used as a reference to calculate relative intensities of the signals observed for each biomolecule detected.

The computer can transform the resulting data into various formats for displaying. In one format, referred to as "spectrum view or retentate map," a standard spectral view can be displayed, wherein the view depicts the quantity of biomolecules reaching the detector at each particular molecular weight. In another format, referred to as "peak map," only the peak height and mass information are retained from the spectrum view, yielding a cleaner image and enabling analytes with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlaid to study subtle changes in relative peak heights. In yet another format, referred to as "difference map view," two or more spectra can be compared, conveniently highlighting unique analytes and analytes which are up- or down-regulated between samples. Biomolecular component mass profiles (spectra) from any two samples may be compared visually. In yet another format, a Spotfire Scatter Plot can be used in which biomolecules that are detected are plotted as a dot in a plot, wherein one axis of the plot represents the apparent molecular weight of the components detected and another axis represents the signal intensity of components detected. For each sample, biomolecules that are detected and the amount of biomolecules present in the sample can be saved in a computer readable medium. This data is then optionally compared to a control (*e*.*g*., a profile or quantity of biomolecules detected in a control).

### IX. SYSTEMS

The present invention also provides systems capable of providing mass spectral profiles of biomolecular components in a sample according to the methods described herein. The system includes one or more adsorbents (*e*.*g*., adsorbents bound to a probe surface, support-bound adsorbents, or the like) capable of capturing biomolecules in the sample under one or more conditions and a gas phase ion spectrometer (*e*.*g*., a mass spectrometer, such as a laser desorption/ionization mass spectrometer) able to profile masses of captured biomolecules under the conditions to provide the mass data. The system also includes a processor (*e*.*g*., in a computer or other logic device) operably connected to the gas phase ion spectrometer. The processor is optionally internal or external to the gas phase ion spectrometer. Optionally, the system includes multiple processors. System software typically includes logic instructions, *e*.*g*., capable of quantifying detected biomolecules, capable of determining closeness-of-fit between one or more detected biomolecule masses in sets of mass data and database entries, or the like.

Figure 5 schematically illustrates surface enhanced laser desorption/ionization time-of-flight mass spectrometry system **700.** As shown, photon energy produced by laser source **702** impacts biochip **704** at surface feature **706,** which includes a selected adsorbent with captured biomolecules. The photon energy causes captured biomolecules at surface feature **706** to desorb and ionize. The desorbed ions are then accelerated through flight tube/mass analyzer **708.** Ions are separated according to mass/charge ratios, which as depicted is simply the mass of the ionic species, because each ion is singly charged. As further illustrated, smaller ions travel faster than larger ions, thereby resolving the species according to mass. Ions produce a detectable signal at detector **710** which signal is processed by information appliance or digital device **712** to generate mass spectrum **714.**

Figure 6 is a schematic showing additional representative details of information appliance **712** from Figure 5 in which various aspects of the present invention may be embodied. As will be understood by practitioners in the art from the teachings provided herein, the invention is optionally implemented in hardware and/or software. In some embodiments, different aspects of the invention are implemented in either client-side logic or server-side logic. As will be understood in the art, the invention or components thereof may be embodied in a media program component (*e*.*g*., a fixed media component) containing logic instructions and/or data that, when loaded into an appropriately configured computing device, cause that device to perform according to the invention. As will also be understood in the art, a fixed media containing logic instructions may be delivered to a viewer on a fixed media for physically loading into a viewer's computer or a fixed media containing logic instructions may reside on a remote server that a viewer accesses through a communication medium in order to download a program component.

Figure 6 shows information appliance or digital device **712** that may be understood as a logical apparatus that can read instructions from media **817** and/or network port **819,** which can optionally be connected to server **820** having fixed media **822.** Apparatus **812** can thereafter use those instructions to direct server or client logic, as understood in the art, to embody aspects of the invention. One type of logical apparatus that may embody the invention is a computer system as illustrated in **712,** containing CPU **807,** optional input devices **809** and **811,** disk drives **815** and optional monitor **805.** Fixed media **817,** or fixed media **822** over port **819,** may be used to program such a system and may represent a disk-type optical or magnetic media, magnetic tape, solid state dynamic or static memory, or the like. In specific embodiments, the invention may be embodied in whole or in part as software recorded on this fixed media. Communication port **819** may also be used to initially receive instructions that are used to program such a system and may represent any type of communication connection. Optionally, the invention is embodied in whole or in part within the circuitry of an application specific integrated circuit (ACIS) or a programmable logic device (PLD). In such a case, the invention may be embodied in a computer understandable descriptor language, which may be used to create an ASIC, or PLD.

### XI. EXPERIMENTAL SECTION

The following experiments are offered only by way of illustration.

### A. MONITORING MAMMALIAN CELL CULTURE MEDIA FOR SECRETED RECOMBINANT TARGET PROTEIN

### 1. OVERVIEW

In overview, this example illustrates various objectives that may be achieved using the methods and devices described herein. In particular, the methods of the invention are optionally performed to analyze several lots or batches of cell culture media for a target polypeptide (*e*.*g*., recombinant polypeptides, naturally occurring polypeptides, *etc*.) secreted by cells using different ProteinChip® arrays, *e*.*g*., to determine which lot has a higher amount of the target polypeptide and lower amount of impurities (*e*.*g*., proteinaceous impurities, *etc*.), to monitor the degradation of the target polypeptide in the different lots, and to monitor modifications to the target polypeptide (*e*.*g*., post-translational modifications). In addition, the methods of the invention are also optionally used to determine the ideal time during a target polypeptide production process at which to harvest cell culture media for maximum recovery of functional target polypeptides.

### 2. RESULTS

The analyses described in this example were performed using a ProteinChip® system (series PBS II), available from Ciphergen Biosystems, Inc. (Fremont, CA), which includes a ProteinChip® reader integrated with ProteinChip® software and a personal computer for analyzing, *e*.*g*., detected polypeptide masses. The ProteinChip® system is capable of detecting biomolecules ranging from less than about 1000 Da up to about 300 kilodaltons or more and calculates the masses based on time-of-flight. The ProteinChip® reader is a laser desorption/ionization time-of-flight mass spectrometer. The ion optics of the ProteinChip® reader are derived from a four-stage, time-lag-focusing ion lens assembly that provides precise, accurate molecular weight determination with excellent mass resolving power. The laser optics have been modified to maximize ion extraction efficiency over the greatest possible sample area, thus increasing analytical sensitivity and reproducibility.

Ciphergen ProteinChip® arrays used in these experiments included the following: an NP (normal phase) chip that comprises silanol functional groups and that binds proteins through hydrophilic interactions; an H4 chip that comprises C16 alkyl functional groups and that binds proteins through hydrophobic interactions; an IMAC (immobilized metal chelate) chip that comprises chelated metal ion functional groups and that binds proteins through coordinate covalent binding; and a SAX (strong anion exchange) chip that comprises ionic functional groups and that binds proteins through electrostatic interactions.

The general protocol used in the analyses described in this example included initially equilibrating the surface features of different ProteinChip® arrays with selected buffers. In particular, hydrophobic (H4) ProteinChip® arrays were equilibrated either with water or 10% acetonitrile, affinity capture (IMAC3-Cu) ProteinChip® arrays were equilibrated with phosphate buffer saline (PBS), and/or anionic exchange (SAX2) ProteinChip® arrays were equilibrated with a Tris-buffer at pH 8.0. Thereafter, 5 µl samples of cell culture media were added to the surface features and the arrays were incubated for 15 minutes. Following incubation, the various array surfaces were washed with the respective equilibration buffers. After a final rinse with water, saturated sinapinic acid (SPA) was added to the different array surface features and bound biomolecular components were analyzed using the PBS II system.

Figure 7A-D are mass spectral traces (abscissa - molecular weight (Daltons); ordinate - relative intensity) showing detected components from cell culture media using H4 and IMAC3-Cu ProteinChip® arrays. Figure 7A shows a mass spectral trace obtained using SELDI from an array exposed to a sample of pure target protein as a control. Figure 7B shows a mass spectral trace obtained using SELDI from an H4 array that had been equilibrated and washed with water. Figure 7C shows a mass spectral trace obtained using SELDI from an H4 array that had been equilibrated and washed with acetonitrile. Figure 7D shows a mass spectral trace obtained using SELDI from an IMAC3-Cu array that had been equilibrated and washed with PBS.

Figures 8A-E are mass spectral traces (abscissa - molecular weight (Daltons); ordinate - relative intensity) showing detected components from different lots of culture media using H4 ProteinChip® arrays. The arrows indicate some of the impurities detected in the various lots. Figure 8A shows a mass spectral trace obtained using SELDI from an array exposed to a sample of pure target protein as a control. Figure 8B shows a mass spectral trace obtained using SELDI from a relatively pure lot. Figure 8C shows a mass spectral trace obtained using SELDI from a lot that included significant numbers of impurities. Figure 8D shows a mass spectral trace obtained using SELDI from the lot having the lowest numbers of impurities. Figure 8E shows a mass spectral trace obtained using SELDI from a lot that included significant numbers of impurities.

Figures 9A-E are mass spectral traces (abscissa - molecular weight (Daltons); ordinate - relative intensity) showing detected components from a quantitative analysis of different lots of culture media using H4 ProteinChip® arrays. Figure A shows a mass spectral trace obtained using SELDI from an array exposed to a sample of pure target protein as a control. Figure 9B shows a mass spectral trace obtained using SELDI from a lot having relatively high amounts of the target protein. Figure 9C shows a mass spectral trace obtained using SELDI from a lot that included relatively low amounts of the target protein. Figure 9D shows a mass spectral trace obtained using SELDI from the lot having the highest amount of the target protein. Figure 9E shows a mass spectral trace obtained using SELDI from a lot having relatively low amounts of the target protein.

From the results presented in this example, it is apparent that the methods of the invention provide significant advantages relative to other analytical techniques. In particular, the SELDI mass spectral profiles generated according to the methods described herein were obtained directly from crude cell culture media. This is typically not feasible for other types of mass spectrometrical analyses. For example, other forms of mass spectrometry generally require that target proteins be purified prior to detecting, *e.g*., the quantity of the target protein secreted into a cell culture medium. In addition, the methods exemplified above used only relatively small sample amounts (*e.g.,* 5 µl) to perform an analysis and results are typically rapidly obtainable, such that an entire assay can be performed in about 30 minutes. For example, other analytical techniques, such as western blotting typically require days before results are obtained. Also, the SELDI-based methods described herein are optionally used to continuously monitor the cell culture media, *e.g*., to identify which batches among a set of production batches to continue processing and which to discard early in the process, thus saving time and resources. In addition, the methods are optionally used to optimize a given production process. For example, the methods of the invention are optionally used to identify points during a cell culture production process at which to supplement the media with essential factors for cell growth (*e.g*., metabolites, growth factors, or the like) when they are decreased, at which to induce target gene expression by adding induction agents to the media, at which to harvest cell culture media to obtain maximum quantities of non-degraded target proteins, or the like. The methods described herein also permit users to monitor various post-translational modifications to the target protein and to monitor target protein purification from cell culture media.

### B. SELDI-ASSISTED PROTEIN MICROPURIFICATION FOR PROTEIN IDENTIFICATION

### 1. CURRENT TECHNOLOGIES

Various strategies exist for identifying proteins in complex samples, including mass spectrometric identification of proteins isolated by 2-D gel electrophoresis. Some mass spectrometric-based strategies are described in detail in, *e.g*., Steven Hall et al., "Mass Spectrometric Identification of Proteins Isolated by Two-dimensional Gel Electrophoresis," at pages 171-202 of Burlingame and Carr (Eds.), Mass Spectrometry in the Biological Sciences, Humana Press (1996)(Hall). To illustrate, Figure 10 provides a flow chart that schematically shows steps in one procedure described in Hall for identifying 2-D PAGE isolated cellular proteins. As shown, components of a cell lysate **(E1)** sample are separated in a 2-D gel **(E2)** and visualized by Coomassie staining/destaining **(E3).** Thereafter, a protein of interest is excised **(E4)**(*e.g*., typically about 100 pmoles of the protein) and electroeluted from the polyacrylamide matrix **(E5).** The electroeluted protein is then acetone precipitated to produce an SDS and stain free protein **(E6).** Following enzymatic digestion of the protein to produce peptides **(E7),** the peptides are separated using microbore HPLC **(E8).** Molecular masses of the peptides are determined by liquid secondary ion mass spectrometry (LSIMS)**(E9)** and the peptides are sequenced by high energy collision-induced dissociation (CID)**(E10).** Proteins are then identified by conducting a database search **(E11).**

Figure 11 is a flow diagram that schematically shows various steps involved in another procedure described in Hall for identifying proteins from 2-D gels. As shown, untreated **(F1)** or treated **(F2)** cells are lysed and cellular proteins are extracted **(F3).** The extracted proteins are then separated and visualized using 2-D PAGE **(F4).** Proteins of interest, typically in the pmole range, are removed from the gel by electroelution or by in-gel digestion **(F5),** and all proteins so removed are enzymatically digested **(F6).** Optionally, peptides are separated using HPLC **(F7)** and the masses of separated peptides are determined by MALDI MS **(F9),** or unseparated in-gel digested proteins are analyzed using MALDI **(F8).** Following step **F8,** peptide masses are, *e.g*., used to search a peptide mass database **(F13).** If the database search is conclusive, the protein is identified **(F16)** and the identification is confirmed by immunoblot analysis **(F17).** If the database search is inconclusive, the peptides of step **F13** are subjected to the same analyses as the peptides of step **F9.** In particular, the peptide masses of step **F10** are distinguished according to mass. Peptides having masses in excess of 2000 Da are sequenced by Edman degradation **(F12),** whereas peptides having masses less than 2000 Da are sequenced by tandem mass spectrometry **(F11).** Peptide sequences determined in steps **F11** and **F12** are then used to search a protein sequence database **(F14).** If the protein is not identified, oligonucleotides are reverse-translated from the protein sequence information are designed for cloning **(F15).** If the protein is identified **(F16),** the identification is confirmed by immunoblot analysis **(F17).**

### 2. PROTEIN PROFILING AND MARKER IDENTIFICATION

The present example illustrates the use of the methods described herein, *e.g.*, for detection of proteins up or down regulated in biological samples, for the identification of protein marker, or the like with the ProteinChip® technology. In particular, the example demonstrates the utility of the invention in mapping differentially expressed proteins using ProteinChip® arrays and size selection spin columns, in developing assays for protein markers, and for phenotypic validation processes. The example further illustrates the utility of the methods described herein in the purification of protein markers using spin columns and arrays available from Ciphergen, Inc. (Fremont, CA) in the enzymatic digestion of purified protein using various types of proteases, in peptide mass determinations using the PBS II system, in database searches to identify proteins, in the partial purification of peptide fragments, in the sequencing of partially purified peptides, and in the design of DNA probes, *e.g*., for use in cloning genes.

Figure 12 is a flow chart that schematically shows steps involved in a protein profiling procedure. As shown in step **G3,** protein profiles are generated for biological sample A (calf serum)**(G1)** and biological sample B (calf serum and protein X (*i.e.,* the target protein marker))**(G2)** using four to five types of ProteinChip® arrays. Optionally, the samples are purified by size-selection spin columns, which generally takes about 15 minutes to perform. Following computer analysis of the protein profiles of samples A and B are compared **(G4).** This data is processed to detect the target protein marker via its molecular weight **(G5).** Based upon the data collected in the preceding steps, an assay is developed for the target protein marker **(G6).** The total time consumed to complete this process is typically about one day.

Figures 13A-C are mass spectral traces (abscissa - molecular weight (Daltons); ordinate - relative intensity) showing detected protein profiles on NP ProteinChip® arrays after being washed and a difference map. Figure 13A shows a mass spectral trace obtained using SELDI of sample A (calf serum) from an NP array that involved a wash. Figure 13B shows a mass spectral trace obtained using SELDI of sample B (calf serum and protein X (*i.e*., the target protein marker)) from an NP array that involved a wash. Detect protein X is indicated on the trace. Figure 13C shows a difference map between the traces shown in Figures 13 A and B. Proteins that are common to both samples A and B are represented by intensity values above zero. The arrow indicates protein X, which is unique to sample B.

Figures 14A-C are mass spectral traces (abscissa - molecular weight (Daltons); ordinate - relative intensity) showing detected protein profiles on NP ProteinChip® arrays after being washed and a difference map. Figure 14A shows a mass spectral trace obtained using SELDI of sample A (calf serum) from an NP array that involved a wash. Figure 14B shows a mass spectral trace obtained using SELDI of sample B (calf serum and protein X (*i.e*., the target protein marker)) from an NP array that involved a wash. Detect protein X is indicated on the trace. Figure 14C shows a difference map between the traces shown in Figures 14 A and B. Proteins that are common to both samples A and B are represented by intensity values above zero. The arrow indicates protein X, which is unique to sample B.

Figure 15 is a flow diagram that schematically shows a general protein fractionation scheme for biological samples. As shown, a biological sample (*e.g*., serum) is fractionated by size **(H1).** Samples are optionally fractionated, *e.g*., using K-30 or K-70 Size Selection Spin Columns. Size-selected protein fractions are isoelectrically fractionated **(H2),** *e.g*., using Q Anion-exchange Spin Columns and different pH and ionic elution buffers. Isoelectrically-selected protein fractions are hydrophobically fractionated **(H3),** *e.g*., using a Hydrophobic Spin Column or a Hydrophobic (H4) ProteinChip® array. SELDI mass spectrometrical analysis of the proteins fractionated by hydrophobic characteristics is performed using a ProteinChip® reader. In step **H4,** the acquired data is analyzed to produce protein profiles and to determine fraction purity. The process typically takes about one to two days to complete.

Figure 16 is a flow diagram that schematically shows various steps in a micropurification of a target protein (protein X) from bovine serum. As shown, a sample of bovine serum that include 0.5% (w/w) of protein X is size fractionated (I1), *e.g*., using a K-70 Size-selection Spin Column, which typically takes about 15 minutes to perform. Size-selected protein fractions are isoelectrically fractionated **(I2),** *e.g.,* using Q Anion-exchange Spin Columns, which typically takes about 30 minutes to perform. Isoelectrically-selected protein fractions are hydrophobically fractionated **(I3),** *e.g*., using a Hydrophobic C8 Spin Column, which typically takes about 15 minutes to perform. SELDI mass spectrometrical analysis of the proteins fractionated by hydrophobic characteristics is performed using a NP and H4 ProteinChip® arrays to produce protein profiles **(I4).** The protein profile data is analyzed to select fractions that include protein X **(I5).** Data analysis typically takes about one hour to perform.

Figures 17A-G are mass spectral traces (abscissa - molecular weight (Daltons); ordinate - relative intensity) showing detected protein profiles from a fractionation assay of bovine serum on size-selection spin columns. Figure 17A shows a mass spectral trace obtained using SELDI of a sample of bovine serum directly. Figure 17B shows a mass spectral trace obtained using SELDI of a first fraction produced using a K-70 Size-selection Spin Column of a sample of bovine serum. Figure 17C shows a mass spectral trace obtained using SELDI of a second fraction produced using a K-70 Size-selection Spin Column of the sample of bovine serum. Defected protein X is indicated. Figure 17D shows a mass spectral trace obtained using SELDI of a third fraction produced using a K-70 Size-selection Spin Column of the sample of bovine serum. Figure 17E shows a mass spectral trace obtained using SELDI of a fourth fraction produced using a K-70 Size-selection Spin Column of the sample of bovine serum. Figure 17F shows a mass spectral trace obtained using SELDI of a fifth fraction produced using a K-70 Size-selection Spin Column of the sample of bovine serum. Figure 17G is a combined plot showing proteins detected in all of the fractions.

Figures 18A-J are mass spectral traces (abscissa - molecular weight (Daltons); ordinate - relative intensity) showing detected protein profiles from a fractionation assay of bovine serum on anion exchange spin columns. Figure 18A shows a mass spectral trace obtained using SELDI of a sample of bovine serum collected from a K-70 Size-selection Spin Column. Figure 18B shows a mass spectral trace obtained using SELDI of a fraction produced using an Anion Exchange Spin Column of a sample of bovine serum eluted with a pH 9.0 buffer. Figure 18C shows a mass spectral trace obtained using SELDI of a fraction produced using an Anion Exchange Spin Column of a sample of bovine serum eluted with a pH 8.0 buffer. Figure 18D shows a mass spectral trace obtained using SELDI of a fraction produced using an Anion Exchange Spin Column of a sample of bovine serum eluted with a pH 7.0 buffer. Figure 18E shows a mass spectral trace obtained using SELDI of a fraction produced using an Anion Exchange Spin Column of a sample of bovine serum eluted with a pH 6.0 buffer. Figure 18F shows a mass spectral trace obtained using SELDI of a fraction produced using an Anion Exchange Spin Column of a sample of bovine serum eluted with a pH 5.0 buffer. Figure 18G shows a mass spectral trace obtained using SELDI of a fraction produced using an Anion Exchange Spin Column of a sample of bovine serum eluted with a pH 4.0 buffer. Figure 18H shows a mass spectral trace obtained using SELDI of a fraction produced using an Anion Exchange Spin Column of a sample of bovine serum eluted with a pH 3.4 buffer. Figure 18I shows a mass spectral trace obtained using SELDI of a fraction produced using an Anion Exchange Spin Column of a sample of bovine serum eluted with a pH 3.4 and 0.2M NaCl buffer. Figure 18J shows a mass spectral trace obtained using SELDI of a fraction produced using an Anion Exchange Spin Column of a sample of bovine serum eluted with a pH 3.4 and 1.0M NaCl buffer.

Figures 19A-D are mass spectral traces (abscissa - molecular weight (Daltons); ordinate - relative intensity) showing a progression of purification of a target protein (protein X) from bovine serum. Figure 19A shows a mass spectral trace obtained using SELDI of an unpurified sample that included bovine serum and protein X. Figure 19B shows a mass spectral trace obtained using SELDI of two combined fractions of a sample of bovine serum and protein X that were produced using a K-70 Size-selection Spin Column. Figure 19C shows a mass spectral trace obtained using SELDI of a sample of bovine serum and protein X that was produced using an Anion Exchange Spin Column in which proteins were eluted with a pH 8.0 buffer. Figure 19D shows a mass spectral trace obtained using SELDI from a hydrophobic ProteinChip® array of a sample of bovine serum and protein X that was washed with 10% acetonitrile.

Figures 20A-C are mass spectral traces (abscissa - molecular weight (Daltons); ordinate - relative intensity) showing purification of a target protein (protein X) on C8 agarose spin columns. Figure 20A shows a mass spectral trace obtained using SELDI of proteins in a fraction of a sample of bovine serum and protein X produced using an Anion-Exchange Spin Column and an elution buffer at pH 8.0. Figure 20B shows a mass spectral trace obtained using SELDI of proteins in a fraction of a sample of bovine serum and protein X produced using a hydrophobic C8 Spin Column and a 2.8M NaCl elution buffer. Purified protein X is indicated. Figure 20C shows a mass spectral trace obtained using SELDI of proteins in a fraction of a sample of bovine serum and protein X produced using an Amicon K10 Concentrator. As shown, significant amounts of protein are lost relative to the analyses illustrated in Figures 20 A and B.

Figures 21A-C are mass spectral traces (abscissa - molecular weight (Daltons); ordinate - relative intensity) of a purified target protein (protein X) digested with V8 (Glu C) endopeptidase in a fingerprinting assay. Figure 21A shows a mass spectral trace obtained using SELDI of V8 only as a control. Figure 21B shows a mass spectral trace obtained using SELDI of purified protein X and V8. Figure 21C shows a mass spectral trace obtained using SELDI of purified protein X, V8, and internal standards.

Figure 22 shows a display screen for a ProFound database search using a purified target protein digested with V8 (Glu C) endopeptidase.

Figures 23A-C are mass spectral traces (abscissa - molecular weight (Daltons); ordinate - relative intensity) showing detected peptide fragments produced by V8 endopeptidase digestion of an HRP sample. Figure 23A shows a mass spectral trace obtained using SELDI of HRP digested by V8 endopeptidase with a PBS II system at a high laser setting. Figure 23B shows a mass spectral trace obtained using SELDI of HRP digested by V8 endopeptidase with a PBS II system at a low laser setting. Figure 23C shows a mass spectral trace obtained using SELDI of HRP digested by V8 endopeptidase with a PBS I system.

Figures 24 A and B are mass spectral traces (abscissa - molecular weight (Daltons); ordinate - relative intensity) showing detected peptide fragments from a tryptic digest of a target protein (protein X) under different conditions. Figure 24A shows a mass spectral trace obtained using SELDI from an H4 ProteinChip® array that involved a water wash prior to detection. Figure 24B shows a mass spectral trace obtained using SELDI from an H4 ProteinChip® array that involved a 30% acetonitrile and 0.1% TFA wash prior to detection.

This example illustrates, *inter alia,* the utility of the methods of the present invention in mapping of purified target proteins (protein X) from fragments of the target, *e.g*., enzymatically produced using V8 (Glu C), trypsin, Lys C, *etc.* and the use of database searches in identifying proteins. Certain advantages which these methods provide include, *e.g*., detecting and purifying target proteins (*e.g*., from complex mixtures, such as serum) in a few days using the SELDI-assisted protein purification technology. In particular, the target protein was micropurified from 30 µl serum to high purity using two kinds of spin columns and a hydrophobic ProteinChip® array or a C8 agarose column. Further, enough protein marker (about 5 pmoles) was purified from 30 microliters of serum for further identification by protein mapping.
The example further illustrates the efficacy of identifying proteins using the PBS II system. Optionally, purified proteins are analyzed by, *e.g*., LC-MS/MS.

### C. METHODS FOR THE RAPID DEVELOPMENT OF PROCESS CHROMATOGRAPHY CONDITIONS UTILIZING SELDI-RETENTATE CHROMATOGRAPHY-MASS SPECTROMETRY

### 1. INTRODUCTION

This example illustrates that protein biochips can be used, e.g., to identify conditions of pH and ionic strength that support selective retention/elution of target proteins and impurity components from ion exchange and other surfaces. Such conditions give corresponding behavior when using process-compatible chromatographic sorbents under elution chromatography conditions. The methods and instrumentation described in this example allow for rapid generation of predictive data for preparative elution chromatography by analyzing the outcome of various protein biochip array experiments that employ a plurality of array surfaces and associated wash conditions. As described below, the retentate chromatography-mass spectrometry (RC-MS) principle was applied to the separation of an Fab antibody fragment expressed in *Escherichia coli* as well as to the separation of recombinant endostatin as expressed in supernatant of *Pichia pastoris* cultures. Determined optimal array binding and elution conditions in terms of ionic strength and pH were directly applied to regular chromatographic columns in step-wise elution mode. Analysis of collected fractions showed favorable correlation to results predicted by the RC-MS method. Accordingly, RC-MS technology rapidly facilitates process chromatography development by providing a facile method to optimize preparative protein separation conditions that consumes minimal sample, while clearly predicting optimal separation conditions for large scale LC purification of proteins from complex biological mixtures.

### 2. MATERIALS AND METHODS

### a) Chemicals and Biologicals

Strong anion exchange (SAX 2), weak cation exchange (WCX 2), hydrophobic (H4), immobilized metal affinity capture (IMAC), and normal phase (NP 2) ProteinChip® arrays used in this study were provided by Ciphergen Biosystems, Inc. (Fremont, CA, USA). Processed arrays were read using a ProteinChip® system (PBS II), laser desorption ionization, time-of-flight mass spectrometer (also available from Ciphergen Biosystems, Inc.).

### b) Simulation of Protein Separation on Arrays

Crude extracts containing expressed target protein from cell culture (Fab fragment and endostatin), were directly deposited upon ProteinChip® array surfaces. Four types of arrays were selected a priori: WCX 2, SAX 2, H4, and IMAC 3, respectively carrying on their surfaces carboxylic acids, quaternary amines, hydrophobic chains, and chelating chemical groups. Each array contained eight distinct spots over which the adsorption of protein could be performed. For WCX 2 surfaces, the pH range investigated was between 4.5 and 6.0. Initially, all spots were equilibrated with 200 µL of a low ionic strength buffer (either a 50 mM acetate buffer or a 20 mM citrate buffer so as to obtain an ionic strength of 5 mS/cm) by using a 96-well bioprocessor (Ciphergen Biosystems, Inc.). Under these conditions, selective protein surface adsorption would be dependent upon the final charge-state of both surface and solvated proteins, with the ultimate objective to preferably adsorb target proteins. After an incubation period of 30 minutes under vigorous shaking, each spot was then washed three times with 200 µL of the appropriate buffer of pH and ionic strength to eliminate weakly or non-adsorbed proteins.

All surfaces were then dried and prepared for SELDI-TOF MS analysis by applying two times 0.8 µL of matrix solution composed of a saturated solution of sinapinic acid in 50% acetonitrile containing 0.5% trifluoroacetic acid. All arrays were then analyzed using a PBS II system. The instrument was used in a positive ion mode, with an ion acceleration potential of 20 kV and a detector gain voltage of 2 kV. The mass range investigated was from 3 to 200 kDa while optimizing time lag focusing conditions at 48 kDa and at 20 kDa for the Fab antibody fragment and endostatin, respectively. This corresponded to respective lag times of 1055 and 1564 nsec. Laser intensity was set between 200 and 280 units according to the sample tested. The instrument was calibrated with bovine serum albumin.

Once the optimal pH for target protein adsorption was determined, a second set of experiments was performed using identical arrays, but this time varying ionic strength, while maintaining constant buffer pH. In this manner, an ideal ionic strength for target protein adsorption with attendant diminution of adsorbed proteins could be determined. Furthermore, the minimal ionic strength required to elute adsorbed target protein would be concomitantly established. The concentration range explored was between zero and 1000 mM sodium chloride in the initial acetate buffer. All samples were loaded as previously described. Each chip surface was then washed three times with 200 µL of buffer of appropriate ionic strength and dried. Arrays were then prepared for SELDI-TOF MS analysis by applying two times 0.8 µL of a saturated solution of sinapinic acid in 50 % acetonitrile containing 0.5 % trifluoroacetic acid and analyzed as previously described.

After this complete set of experiments, the best conditions of pH and ionic strength were identified for target protein adsorption and elution from a resin packed LC column carrying the same functional groups (weak cation exchange: carboxymethyl). For SAX 2 surfaces, the investigation was operated in a similar way, however, the pH range explored was the one generally used for anion exchange chromatography, namely, between 7.5 and 9 using a 50 mM Tris-HCl buffer. The ionic strength was maintained at 5 mS/cm.

Hydrophobic H4 array surfaces were used according to the rules of hydrophobic interaction chromatography. Two sodium chloride concentrations (1 M and 1.5 M) and 4 different pH values (4.5, 6.0, 7.5 and 9.0) were used to promote - adsorption of proteins. After equilibration of each spot with 200 µL of corresponding buffer, 50 µL of crude sample previously adjusted to equilibration pH and ionic strength conditions was incubated for 30 minutes under vigorous shaking. Each spot was then washed three times with 200 µL of buffer at the same pH while varying ionic strength between zero and 1 M sodium chloride. All surfaces were then rapidly washed in deionized water in order to eliminate salts, and then dried and prepared for SELDI-TOF MS analysis by applying matrix solution as described above.

IMAC 3 arrays were investigated using two different metal ions: copper and nickel. Surface spots were first loaded with 50 µL of a 100 mM solution of either copper sulfate or nickel sulfate. Excess metal ions were removed using a quick wash with 200 µL of deionized water. Spots were then equilibrated using 200 µL of 20 mM sodium phosphate buffer at pH 7.0, containing either 500 or 1000 mM sodium chloride. Fifty µL of sample previously adjusted to the same ionic strength was then incubated for 30 minutes under vigorous agitation. Spots were then rapidly washed with deionized water, dried and prepared for SELDI-TOF MS analysis previously described.

### c) Liquid Chromatography

Column LC separation of target proteins from *E. coli* extract and *Pichia pastoris* culture supernatant was performed on CM Zirconia beads (BioSepra, Cergy, France). This choice was dictated by the results obtained from array surface investigations. Columns of 0.3 cm I.D. x 10 cm were first equilibrated with an adsorption buffer of ionic strength and pH determined by preliminary experiments using arrays systems (described above). Feedstocks were directly loaded after filtration through a 0.45 µm membrane. Elution was performed using sodium chloride concentration steps according to information obtained from the preliminary RC-MS experiments. Finally, the sorbent was regenerated by a wash with 5 column volumes of 1M sodium hydroxide. Chromatography separations were accomplished at a linear flow rate of 300 cm/h.

For the Fab fragment separation from *E. coli* extract, conditions of adsorption were met using a 50 mM acetate, 5 mM citrate buffer at pH 4.6 and elution was performed by increasing the ionic strength to 150 mS/cm using sodium chloride in the same buffer. Feedstock volume loaded was 23 ml. For recombinant endostatin from *Pichia pastoris* culture supernatant, adsorption was performed in a 50 mM acetate buffer, pH 5, and elution was performed by increasing the ionic strength up to 800 mM sodium chloride. Feedstock volume loaded was 80 ml. Fractions were then collected and analyzed either by SELDI-TOF MS using a NP 2 ProteinChip® array, or regular SDS polyacrylamide gel electrophoresis.

### d) SDS Polyacrylamide Gel Electrophoresis

Electrophoresis of chromatography fractions was performed on a Mini-PROTEAN 3™ system (BioRad Laboratories, Ivry sur Seine, France) in classical conditions using 15-well pre-casted polyacrylamide plates of 12 or 18% concentration. Samples were prepared by a two-fold dilution in Laemli sample buffer. Twelve µL of sample were loaded per lane, and electrophoretic migration was performed using a tension of 200 volts for 45 minutes. Staining was achieved using coomasssie blue solution in ethanol and acetic acid for 1 hour to 1.5 hours under gentle agitation. Destaining was performed using 40% ethanol, and 10% acetic acid in water.

### 3. RESULTS AND DISCUSSION

Ion-exchange adsorption-desorption mechanisms of proteins to a porous planar surface or to a porous bead are essentially the same. The basic principle of separating proteins from crude mixtures using protein biochip ion exchange surfaces is schematically illustrated in Figures 25 A and B. As per conventional column chromatography, the sample is loaded on the surface of the array in appropriate conditions of pH and ionic strength to capture the protein of interest to the solid support. A wash then follows to eliminate impurities while retaining the target protein on the array. Desorption of unwanted proteins is typically accomplished by increasing the ionic strength of the buffer or varying buffer pH. Contrary to chromatographic separations where mobile phase elutropic strength is designed to elute all proteins for subsequent down-stream or off-line analysis, here retained proteins are ultimately studied. During the final stage of sample preparation, a matrix solution is added that functions to desorb adsorbed proteins from the array and entrain them in growing crystals. The crystals are irradiated by a focused pulse of laser light, which subsequently causes a phase transition, creating gaseous ions that are analyzed in the TOF MS. In this fashion, a mass spectrum can be generated, often indicating whether the protein of interest is adsorbed while additionally informing on the presence of impurities. The lower the number of impurities, the higher the selectivity of the array surfaces for the target protein in the conditions of exploitation.

### a) Fab Antibody Fragment Separation

This analysis was performed with a crude extract of *E. coli* in which a recombinant Fab antibody fragment (47 kDa) was expressed. The total amount of protein present was 700 µg/ml. When using the WCX2 surface (weak cation exchanger) the Fab fragment was effectively adsorbed at a pH range between 4.5 and 4.8. Above this last value, the Fab fragment was not present among surface adsorbed species. However, a major 45 kDa impurity was retained (see, Figure 26A). At pH 5 no proteins at all were present indicating that none of the proteins from the sample were retained. Eventually optimal pH for Fab fragment retention was determined to be between 4.5 and 4.7. For all subsequent array experiments, buffer pH value was thus fixed at 4.6, while experiments proceeded examining the effect of ionic strength by varying the concentration of sodium chloride from 0 - 150 mM in the acetate buffer.

SELDI-TOF MS data from WCX 2 arrays indicated that the Fab fragment was still present at 75 mM sodium chloride, but that it disappeared when the concentration of sodium chloride was equal to or above 150 mM (see, Figure 26B). These results clearly indicated that the Fab fragment could be properly adsorbed on a carboxyl-containing surface at a pH of about 4.6-4.7 and at low ionic strength. Desorption would occur, if the concentration of sodium chloride were increased to at least 150 mM. Since the ionic strength of the crude extract was 10 mS/cm, equivalent to a sodium chloride concentration of 50 mM in 50 mM acetate/5 mM citrate buffer, no changes would have to be made to get the Fab fragment adsorbed.

Based upon the ProteinChip® array results, a column LC separation was performed using a sorbent of similar composition to that of the array's surface. The sorbent, CM Zirconia, was packed in a chromatography column of 0.3 cm I.D. x 10 cm. Adsorption and washing buffer was 50 mM acetate/5mM citrate buffer, containing 25 mM sodium chloride, pH 4.6 equivalent to an ionic strength of about 8 mS/cm and elution was accomplished at the same pH, but increasing the sodium concentration to 150 mM. Figure 27 shows the chromatographic separation profile with clear indication of an eluted peak. An analysis of the eluted fractions by mass spectrometry showed that it contained mostly Fab fragment with some impurities of lower molecular mass (see, Figure 28). A major impurity present had a mass close to 37 kDa. Polyacrylamide gel electrophoresis analysis further confirmed the presence of the Fab fragment. As a whole, the Fab fragment was adequately captured from crude feedstock and was effectively concentrated by 10-fold while simultaneously eliminating many impurities. Further purification of the Fab fraction would require a secondary, orthogonal separation scheme.

An analysis of the crude extract at different pH and ionic strengths similar to the one described for WCX2 arrays was performed using SAX2 array surfaces. Regardless of ionic strength, the Fab fragment was not retained. As such, a SAX2 surface would provide a useful, orthogonal separation scheme to the cation exchange resin, further purifying the Fab fraction by adsorbing impurities in lieu of target protein. This strong anion exchange column purification step was performed using a Q Ceramic HyperD® F sorbent equilibrated with a 50 mM Tris-HCl buffer at pH 7.0 (column dimensions: 0.66 cm ID x 10 cm). Three hundred µL of eluted fraction from CM Zirconia column was diluted 10 times in Tris-HCl buffer at pH 7.0 in order to decrease the ionic strength to a value compatible with the column initial conditions (lower than 15 mS/cm). Under these conditions, the Fab fragment was effectively found in the flow-through. Analysis of collected fractions from the Q resin separation showed in fact that the purity of the Fab fragment was significantly enhanced, however, minor impurities were still present (see, Figure 28). This experiment unambiguously demonstrated the effectiveness of using ProteinChip® arrays to predict conditions for liquid chromatography.

### b) Recombinant endostatin purification

To confirm that the RC-MS approach could be extended to other proteins, a second set of experiments was performed using a culture supernatant of *Pichia pastoris* containing recombinant endostatin, a 20.1 kDa protein inhibiting endothelial cell proliferation. The total protein concentration of the feedstock was 0.6 mg/ml, while containing an endostatin concentration of 0.05 mg/ml. The crude extract was tested on four types of array surfaces as previously described: weak cation exchange, strong anion exchange, hydrophobic, and chelating surfaces with copper. After loading, array surfaces were washed with appropriate buffers to mimic adsorption chromatography, prior to MS analysis. As shown in Figures 29A-D, it appeared that WCX 2 arrays (Figure 29A) adsorbed endostatin along with minor impurities, whereas SAX 2, H4, and IMAC3 arrays (Figures 29B-C, respectively) did not show any significant interaction with the target recombinant protein. In particular, the SAX array surface did not adsorb proteins at all, while H4 and IMAC 3 array surfaces showed different adsorption behavior. Both H4 and IMAC 3 surfaces unambiguously interacted with other protein species excluding endostatin. H4 arrays adsorbed mainly a protein of a molecular mass close to 25 kDa, while IMAC3 surfaces bound a protein of lower molecular weight (17 kDa).

In order to define conditions to purify endostatin using cation exchange chromatography, array adsorption/desorption conditions were optimized using a narrow pH range: 5.0, 5.5, 6.0, and 6.5 (Figure 30A). Afterwards, different ionic strengths were tested to identify a sodium chloride concentration capable of annihilating endostatin surface interaction (Figure 30B). It was found that pH 5.0 promoted optimal endostatin surface interaction. No modification of original ionic strength was necessary. As expected, an increase in sodium chloride concentration clearly promoted endostatin desorption from WCX2 surfaces starting at about 200 mM of NaCl. MS analysis indicated that full desorption of endostatin was effected at NaCl concentrations greater than about 400 mM.

From these defined conditions, a column of CM Zirconia was prepared and used in a preparative manner. After equilibration, 80 ml of cell culture supernatant was adjusted to pH 5.0 and was loaded onto the column. Elution of endostatin was accomplished by a two-step concentration gradient of sodium chloride: 200 mM followed by 800 mM at pH 5.0. For additional details see the Experimental section above and Figure 31A. Analysis of collected fractions evidenced that all endostatin was adsorbed when the column was loaded and washed; elution of endostatin occurred essentially after the second increase of sodium chloride concentration.

Electrophoresis (see, Figure 31B) revealed that endostatin was totally eluted using a sodium chloride buffer concentration of 800 mM. Analysis of eluted fractions indicated that overall endostatin purity was, in all cases, significantly increased compared to the initial feedstock composition (lane "1"). Impurities of lower molecular mass than endostatin were present in trace amounts. According to electrophoresis results, purity of recombinant endostatin improved from less than 10% to at least 90% in a single step.

Considering that Cu⁺² IMAC arrays exclusively adsorbed a 17 kDa protein impurity, which was still present in trace amount after CM Zirconia chromatography (lane 3 of Figure 31B), it is suggested that a polishing column of chelating beads complexed with copper could be used to further endostatin fraction purity. Conversely, as suggested by preliminary results obtained using H4 array surfaces (Figure 29), impurities with lower electrophoretic mobility (larger molecular weight) would be removed in a second polishing separation step using hydrophobic interaction chromatography.

### 4. CONCLUSION

This work has demonstrated the ability of the RC-MS method to rapidly predict effective preparative separation conditions in short order while consuming minimal sample. The results of this study indicate that RC-MS should be applicable to purify many other biological liquids and tissue extracts. To begin with, the process merely requires a priori knowledge of target protein molecular weight. Alternatively, target protein molecular weight may be easily determined using the SELDI -TOF MS approach. Serial, multicolumn process separation schemes could also be designed. Although composite data from initial array operations provide enough information about the behavior of a target protein and impurities to design an entire uni-dimensional LC separation scheme, it may be also useful to start a second array analysis of the eluted fraction from the first column, to further gain insight into the nature of impurities and provide potential polishing strategies. Moreover, impurity tracking from separated fractions becomes possible with a very good level of sensitivity. Indeed, detection of species is possible in the femtomole range.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above may be used in various combinations.

## Claims

1. A method of monitoring purification of a target polypeptide from a mixture, the method comprising:
(a) generating a first surface enhanced laser desorption/ionization mass spectral profile of biomolecular components in a mixture that comprises the target polypeptide and at least one contaminating biomolecule, wherein the first profile provides qualitative or quantitative detection of biomolecular components in the mixture;
(b) subjecting the target polypeptide to a purification process by removing at least a portion of at least one contaminating biomolecule from the mixture, thereby providing a purer mixture comprising the target polypeptide;
(c) generating a second surface enhanced laser desorption/ionization mass spectral profile of biomolecular components in the purer mixture; and
(d) comparing the first and second profiles to determine a qualitative or quantitative difference between biomolecular components in the mixture and the purer mixture, thereby monitoring the purification of the target polypeptide.

2. The method of claim 1, wherein (d) comprises:
- determining a quantitative difference in purity of the target polypeptide in the mixture and the purer mixture, wherein purity is a measure of relative amounts of the target polypeptide and the at least one contaminating biomolecule;
- determining a qualitative difference between the target polypeptide in the mixture and in the purer mixture, wherein the qualitative difference is a measure of the target polypeptide and degraded forms of the target polypeptide; or
- determining a quantitative or qualitative difference between contaminating bimolecular components in the first and second profiles.

3. The method of claim 1, wherein (d) comprises determining a qualitative difference in the target polypeptide between the mixtures, wherein the qualitative difference comprises a difference in the target polypeptide and a modified form of the target polypeptide, wherein the modification is glycosylation, phosphorylation, lipidation, enzymatic breakdown, labeling, or polypeptide aggregation.

4. The method of claim 3, wherein the modification occurs in vivo or in vitro.

5. The method of any one of claims 1 to 4, further comprising identifying one or more contaminating biomolecules in the mixture or the purer mixture.

6. The method of any one of claims 1 to 5, wherein the purification process is electrophoresis, chromatography, precipitation, dialysis, filtration, or centrifugation.

7. The method of claim 6, wherein the chromatography process is affinity chromatography, high performance liquid chromatography, ion exchange chromatography, hydrophobic interaction chromatography or size exclusion chromatography.

8. The method of any one of claims 1 to 7, further comprising:
(e) subjecting the target polypeptide to at least additional purification process thereby providing at least one subsequent mixture;
(f) generating a subsequent surface enhanced laser desorption/ionization mass spectral profile of biomolecular components in the at least one subsequent mixture; and
(g) comparing the subsequent surface enhanced laser desorption/ionization mass spectral profile to another surface enhanced laser desorption/ionization mass spectral profile to determine a qualitative or quantitative difference between biomolecular components in the subsequent mixture and another mixture.

9. The method of any one of claims 1 to 8, wherein the mixture comprises a cell culture medium from which cells have been removed, which cell culture medium comprises the target polypeptide, or wherein the mixture comprises a cell lysate.

10. A method of purifying a target polypeptide, the method comprising:
(a) identifying purification conditions by:
(i) contacting a mixture comprising the target polypeptide with a plurality of substrate-bound adsorbents;
(ii) washing each of the adsorbents with a different eluant to allow selective binding of polypeptides in the mixture to the adsorbents;
(iii) generating a surface enhanced laser desorption/ionization mass spectral profile of biomolecular components in the mixture, wherein a surface enhanced laser desorption/ionization mass spectral profile provides qualitative or quantitative detection of biomolecular components in the mixture; and
(iv) identifying (1) a wash condition under which the target polypeptide is adsorbed to the adsorbent and contaminating polypeptides are eluted from the adsorbent and (2) a wash condition under which the target polypeptide is eluted from the adsorbent;
(b) contacting a batch of the mixture with a chromatographic medium comprising the adsorbent and washing the chromatographic medium with an identified wash condition under which the target polypeptide is adsorbed to the adsorbent and contaminating polypeptides are eluted from the adsorbent;
(c) washing the chromatographic medium with an identified wash condition under which the target polypeptide is eluted from the adsorbent; and
(d) collecting the eluted target polypeptide.

11. The method of claim 10, wherein the batch comprises at least 1000 liters.

12. The method of claim 10 or 11, wherein the substrate-bound adsorbents are in the form of a probe, or wherein the adsorbents comprise chromatographic or biospecific adsorbents.

13. The method of any one of claims 10 to 12, wherein the wash conditions comprise different parameters selected from: a salt concentration; a detergent concentration; a pH; a buffering capacity; an ionic strength; a water structure characteristic; a detergent type; a hydrophobicity; a dielectric constant; a concentration of at least one solvent; and a concentration of at least one solute.

14. The method of any one of the preceding claims, wherein the target polypeptide is a hormone, cytokine, immunoglobulin, enzyme, receptor, antigen, regulation factor or protein carrier.

15. The method of any one of the preceding claims, wherein the target polypeptide is a recombinant polypeptide or a naturally occurring polypeptide.

16. The method of any one of the preceding claims, wherein the biomolecular component is a growth factor, induction agent or metabolite.

## Patentansprüche

1. Verfahren zur Überwachung der Reinigung eines Zielpolypeptids aus einem Gemisch, wobei das Verfahren umfasst:
(a) Erzeugen eines ersten oberflächenverstärkten Laser-Desorptions/ lonisierungs-massenspektrometrischen Profils von biomolekularen Komponenten in einem Gemisch, das das Zielpolypeptid und mindestens ein verunreinigendes Biomolekül umfasst, wobei das erste Profil einen qualitativen oder quantitativen Nachweis biomolekularer Komponenten in dem Gemisch liefert;
(b) das Zielpolypeptid einem Reinigungsprozess unterziehen durch Entfernen von mindestens einem Teil des mindestens einen verunreinigenden Biomoleküls aus dem Gemisch, wodurch ein reineres Gemisch bereitgestellt wird, das das Polypeptid umfasst;
(c) Erzeugen eines zweiten oberflächenverstärkten Laser-Desorptions/ Ionisierungs-massenspektrometrischen Profils von biomolekularen Komponenten in dem reineren Gemisch; und
(d) Vergleichen von erstem und zweitem Profil, um einen qualitativen oder quantitativen Unterschied an biomolekularen Komponenten in dem Gemisch und dem reineren Gemisch festzustellen, wodurch die Reinigung des Zielpolypeptid überwacht wird.

2. Verfahren nach Anspruch 1, wobei (d) umfasst:
- Bestimmen eines quantitativen Unterschieds an Reinheit des Zielpolypeptids in dem Gemisch und dem reineren Gemisch, wobei Reinheit ein Maß der relativen Mengen des Zielpolypeptids und des mindestens einen verunreinigenden Biomoleküls ist;
- Bestimmen eines qualitativen Unterschieds an Zielpolypeptid in dem Gemisch und in dem reineren Gemisch, wobei der qualitative Unterschied ein Maß des Zielpolypeptids und von Abbauformen des Zielpolypeptids ist; oder
- Bestimmen eines quantitativen oder qualitativen Unterschieds an kontaminierenden biomolekularen Komponenten im ersten und zweiten Profil.

3. Verfahren nach Anspruch 1, wobei (d) das Bestimmen eines qualitativen Unterschieds an Zielpolypeptid in den Gemischen umfasst, wobei der qualitative Unterschied einen Unterschied an Zielpolypeptid und einer modifizierten Form des Zielpolypeptids umfasst, wobei die Modifikation eine Glykosylierung, Phosphorylierung, Lipidbindung, Markierung, Polypeptidaggregation oder ein enzymatischer Abbau ist.

4. Verfahren nach Anspruch 3, wobei die Modifikation *in vivo* oder *in vitro* erfolgt.

5. Verfahren nach einem der Ansprüche 1 bis 4, das ferner ein Identifizieren eines oder mehrerer verunreinigender Biomoleküle in dem Gemisch oder dem reineren Gemisch umfasst

6. Verfahren nach einem der Ansprüche 1 bis 5, wobei der Reinigungsprozess eine Elektrophorese, Chromatographie, Präzipitation, Dialyse, Filtration oder Zentrifugation ist.

7. Verfahren nach Anspruch 6, wobei der Chromatographieprozess eine Affinitätschromatographie, Hochleistungs-Flüssigchromatographie, Ionenaustauschchromatographie, hydrophobe Wechselwirkungschromatographie oder Größenausschluss-Chromatographie ist.

8. Verfahren nach einem der Ansprüche 1 bis 7, ferner umfassend:
(e) das Zielpolypeptid mindestens einem weiteren Reinigungsprozess unterziehen, wodurch mindestens ein Folgegemisch bereitgestellt wird;
(f) Erzeugen eines oberflächenverstärkten Laser-Desorptions/Ionisierungs-massenspektrometrischen Folgeprofils von biomolekularen Komponenten in dem mindestens einen Folgegemisch; und
(g) Vergleichen des oberflächenverstärkten Laser-Desorptions/Ionisierungs-massenspektrometrischen Folgeprofils mit einem weiteren oberflächenverstärkten Laser-Desorptions/Ionisierungs-massenspektrometrischen Profil, um einen qualitativen oder quantitativen Unterschied an biomolekularen Komponenten in dem Folgegemisch und dem anderen Gemisch festzustellen.

9. Verfahren nach einem der Ansprüche 1 bis 8, wobei das Gemisch ein Zellkulturmedium umfasst, aus dem Zellen entfernt wurden, wobei dieses Zellkulturmedium das Zielpolypeptid umfasst, oder wobei das Gemisch ein Zelllysat umfasst.

10. Verfahren zur Reinigung eines Zielpolypeptids, wobei das Verfahren umfasst:
(a) Identifizieren von Reinigungsbedingungen mittels:
(i) Inkontaktbringen eines Gemisches, das das Zielpolypeptid umfasst, mit einer Mehrzahl von substratgebundenen adsorbierenden Stoffen;
(ii) Waschen jedes der adsorbierenden Stoffe mit einem unterschiedlichen Eluationsmittel, um eine selektive Bindung von Polypeptiden im Gemisch an die adsorbierenden Stoffe zu ermöglichen;
(iii) Erzeugen eines oberflächenverstärkten Laser-Desorptions/ Ionisierungs-massenspektrometrischen Profils von biomolekularen Komponenten im Gemisch, wobei ein oberflächenverstärktes Laser-Desorptions/Ionisierungs-massenspektrometrisches Profil einen qualitativen oder quantitativen Nachweis von biomolekularen Komponenten im Gemisch liefert; und
(iv) Identifizieren (1) einer Waschbedingung, unter der das Zielpolypeptid an den adsorbierende Stoff adsorbiert wird und verunreinigende Polypeptide von dem adsorbierenden Stoff eluiert werden, und (2) eine Waschbedingung, unter der das Zielpolypeptid von dem adsorbierenden Stoff eluiert wird;
(b) Inkontaktbringen einer Charge des Gemisches mit einem Chromatographiemedium, das den adsorbierenden Stoff umfasst, und Waschen des Chromatographiemediums bei einer identifizierten Waschbedingung, unter der das Zielpolypeptid an den adsorbierenden Stoff adsorbiert wird und verunreinigende Polypeptide von dem adsorbierenden Stoff eluiert werden;
(c) Waschen des Chromatographiemediums bei einer identifizierten Waschbedingung, unter der das Zielpolypeptid von der adsorbierenden Stoff eluiert wird; und
(d) Sammeln des eluierten Zielpolypeptids.

11. Verfahren nach Anspruch 10, wobei die Charge mindestens 1000 Liter umfasst.

12. Verfahren nach Anspruch 10 oder 11, wobei die substratgebundenen adsorbierenden Stoffe in Form einer Sonde vorliegen, oder wobei die adsorbierenden Substanzen chromatographische oder biospezifische adsorbierende Stoffe umfassen.

13. Verfahren nach einem der Ansprüche 10 bis 12, wobei die Waschbedingungen unterschiedliche Parameter umfassen, die ausgewählt sind aus: einer Salzkonzentration; einer Detergenzkonzentration; einem pH-Wert; einer Pufferkapazität; einer Ionenstärke; einer Wasserstruktureigenschaft; einer Detergenzart; einer Hydrophobizität; einer dielektrischen Konstante; einer Konzentration mindestens eines Lösungsmittels; und einer Konzentration von mindestens einem gelösten Stoff.

14. Verfahren nach einem der vorangehenden Ansprüche, wobei das Zielpolypeptid ein Hormon, Cytokin, Immunglobulin, Enzym, Rezeptor, Antigen, Regulationsfaktor oder Proteinträger ist.

15. Verfahren nach einem der vorangehenden Ansprüche, wobei das Zielpolypeptid ein rekombinantes Polypeptid oder ein natürlich vorkommendes Polypeptid ist.

16. Verfahren nach einem der vorangehenden Ansprüche, wobei die biomolekulare Komponente ein Wachstumsfaktor, Induktionsmittel oder Stoffwechselprodukt ist.

## Revendications

1. Procédé pour contrôler la purification d'un polypeptide cible à partir d'un mésange, le procédé comprenant les étapes de :
(a) générer un premier profil spectral de masse à désorption/ionisation de surface par impact laser améliorée de composants biomoléculaires dans un mélange qui comprend le polypeptide cible et au moins une biomolécule contaminante, dans lequel le premier profil fournit une détection qualitative et quantitative des composants biomoléculaires dans le mélange ;
(b) soumettre le polypeptide cible à un procédé de purification en ôtant au moins une portion d'au moins une biomolécule contaminante du mélange, produisant de ce fait un mélange plus pur comprenant le polypeptide cible ;
(c) générer un second profil spectral de masse à désorption/ionisation de surface par impact laser améliorée de composants biomoléculaires dans le mélange plus pur ; et
(d) comparer les premier et second profils pour déterminer une différence qualitative ou quantitative entre les composants biomoléculaires dans le mélange et le mélange plus pur, et de ce fait contrôler la purification du polypeptide cible.

2. Procédé selon la revendication 1, dans lequel l'étape (d) comprend :
- le fait de déterminer une différence quantitative de pureté du polypeptide cible entre le mélange et le mélange plus pur, dans lequel la pureté est une mesure des quantités relatives du polypeptide cible et d'au moins d'une biomolécule contaminante ;
- le fait de déterminer une différence qualitative du polypeptide cible entre le mélange et le mélange plus pur, dans lequel la différence qualitative est la mesure du polypeptide cible et des formes dégradées du polypeptide cible ; ou
- le fait de déterminer une différence quantitative ou qualitative dans les composants biomoléculaires contaminants entre les premier et second profils.

3. Procédé selon la revendication 1, dans lequel l'étape (d) comprend le fait de déterminer une différence qualitative dans le polypeptide cible entre les mélanges, procédé dans lequel la différence qualitative comprend une différence entre le polypeptide cible et la forme modifiée du polypeptide cible, la modification étant une glycosylation, une phosphorylation, une lipidation, une transformation enzymatique, un marquage ou un agrégat de polypeptide.

4. Procédé selon la revendication 3, dans lequel la modification s'effectue *in vivo* ou *in vitro*.

5. Procédé selon l'une quelconque des revendications 1 à 4, qui comprend en outre le fait d'identifier une ou plusieurs biomolécules contaminantes dans le mélange ou le mélange plus pur.

6. Procédé selon l'une quelconque des revendications 1 à 5, dans lequel le procédé de purification est l'électrophorèse, la chromatographie, la précipitation, la dialyse, la filtration ou la centrifugation.

7. Procédé selon la revendication 6, dans lequel le procédé de chromatographie est la chromatographie d'affinité, la chromatographie liquide à haute performance, la chromatographie d'échange d'ions, la chromatographie d'interaction hydrophobe ou la chromatographie d'exclusion de taille.

8. Procédé selon l'une quelconque des revendications 1 à 7, qui comprend en outre :
(e) le fait de soumettre le polypeptide cible à au moins un procédé de purification additionnel, fournissant au moins un mélange supplémentaire ;
(f) le fait de générer un profil spectral supplémentaire de masse à désorption/ionisation de surface par impact laser améliorée des composants biomoléculaires dans au moins un mélange supplémentaire ; et
(g) le fait de comparer le profil spectral de masse supplémentaire à désorption/ionisation de surface par impact laser améliorée à un autre profil spectral de masse à désorption/ionisation de surface par impact laser améliorée pour déterminer une différence qualitative ou quantitative dans les composants biomoléculaires entre le mélange supplémentaire et un autre mélange.

9. Procédé selon l'une quelconque des revendications 1 à 8, dans lequel le mélange comprend un milieu de culture cellulaire dont les cellules ont été ôtées, ledit milieu de culture cellulaire comprenant le polypeptide cible, ou procédé dans lequel le mélange comprend un lysat cellulaire.

10. Procédé de purification d'un polypeptide cible, le procédé comprenant :
(a) le fait d'identifier les conditions de purification par :
(i) le fait de mettre en contact un mélange comprenant le polypeptide cible avec un ensemble d'adsorbants liés au substrat ;
(ii) le fait de laver chacun de ces adsorbants avec un éluant différent pour permettre la fixation sélective des polypeptides aux adsorbants dans le mélange ;
(iii) le fait de générer un profil spectral de masse à désorption/ionisation de surface par impact laser améliorée de composants biomoléculaires dans le mélange, procédé dans lequel un profil spectral de masse à désorption/ionisation de surface par impact laser améliorée fournit une détection qualitative ou quantitative des composants biomoléculaires dans le mélange ; et
(iv) le fait d'identifier (1) une condition de lavage dans laquelle le polypeptide cible est adsorbé à l'adsorbant et les polypeptides contaminants sont élués de l'adsorbant et (2) une condition de lavage dans laquelle le polypeptide cible est élué de l'adsorbant ;
(b) le fait de mettre en contact un lot du mélange avec un milieu chromatographique comprenant l'adsorbant et le fait de laver le milieu chromatographique en utilisant une condition de lavage identifiée dans laquelle le polypeptide cible est adsorbé à l'adsorbant et les polypeptides contaminants sont élués de l'adsorbant ;
(c) le fait de laver le milieu chromatographique en utilisant une condition de lavage identifiée dans laquelle le polypeptide cible est élué de l'adsorbant ; et
(d) le fait de récolter le polypeptide cible élué.

11. Procédé selon la revendication 10, dans lequel le lot comprend au moins 1000 litres.

12. Procédé selon la revendication 10 ou 11, dans lequel les adsorbants liés au substrat sont sous la forme d'une sonde, ou procédé dans lequel les adsorbants comprennent des adsorbants chromatographiques ou biospécifiques.

13. Procédé selon l'une quelconque des revendications 10 à 12, dans lequel les conditions de lavage comprennent des paramètres différents choisis parmi : la concentration en sel, la concentration en détergent, le pH, la capacité de tampon, la force ionique, les caractéristiques structurelles de l'eau, le type de détergent, l'hydrophobicité, la constante diélectrique, la concentration d'au moins un solvant, et la concentration d'au moins un soluté.

14. Procédé selon l'une quelconque des revendications précédentes, dans lequel le polypeptide cible est une hormone, une cytokine, une immunoglobuline, une enzyme, un récepteur, un antigène, un facteur de régulation ou un transporteur de protéine.

15. Procédé selon l'une quelconque des revendications précédentes, dans lequel le polypeptide cible est un polypeptide recombinant ou un polypeptide naturel.

16. Procédé selon l'une quelconque des revendications précédentes, dans lequel le composant biomoléculaire est un facteur de croissance, un agent d'induction ou un métabolite.
